# EUROPÄISCHE PATENTSCHRIFT

(11) **EP 2 941 429 B1**
(45) Veröffentlichungstag und Bekanntmachung des Hinweises auf die Patenterteilung: **25.01.2017**
(21) Anmeldenummer: 14781129.3
(22) Anmeldetag: 29.09.2014
(51) Int. Cl.: C07D 471/04, A61K 31/437

(54) **FLUOR-9-METHYL-BETA-CARBOLINE**
FLUOR-9-METHYL-BETA-CARBOLINES
FLUORO-9-MÉTHYL-BETA-CARBOLINES

(30) Priorität: 29.09.2013 EP 13186560
(43) Veröffentlichungstag der Anmeldung: 11.11.2015
(73) Patentinhaber: Audiocure Pharma GmbH, 10115 Berlin (DE)
(72) Erfinder: ROMMELSPACHER, Hans, 14193 Berlin (DE); ENZENSPERGER, Christoph, 07745 Jena (DE)
(74) Vertreter: Arth, Hans-Lothar
(86) Internationale Anmeldenummer: PCT/EP2014/070840
(87) Internationale Veröffentlichungsnummer: WO 2015/044434

(56) Entgegenhaltungen:
- WO-A1-2011/079841
- US-A1- 2004 038 970
- CASTRO A C ET AL: "NOVEL IKK INHIBITORS: BETA-CARBOLINES", BIOORGANIC & MEDICINAL CHEMISTRY LETTERS, PERGAMON, GB, Bd. 13, Nr. 14, 2003, Seiten 2419-2422, XP002295562, ISSN: 0960-894X, DOI: 10.1016/S0960-894X(03)00408-6

### SEQUENCE LISTING

<110> Audiocure Pharma GmbH
<120> Fluor-9-methyl-ß-carboline
<130> AUD-P03632WO
<160> 22
<170> PatentIn version 3.5
<210> 1
   <211> 18
   <212> DNA
   <213> Artificial Sequence
<220>
   <223> Forward Primer für BDNF
<400> 1
   ggatgaggac cagaaagt 18
<210> 2
   <211> 19
   <212> DNA
   <213> Artificial Sequence
<220>
   <223> Reverse Primer für BDNF
<400> 2
   agcagaaaga gaagaggag 19
<210> 3
   <211> 22
   <212> DNA
   <213> Artificial Sequence
<220>
   <223> Forward Primer für Lif
<400> 3
   ccaacaacct ggacaagcta tg 22
<210> 4
   <211> 20
   <212> DNA
   <213> Artificial Sequence
<220>
   <223> Reverse Primer für Lif
<400> 4
   gtggcgttga gcttgctgtg 20
<210> 5
   <211> 18
   <212> DNA
   <213> Artificial Sequence
<220>
   <223> Forward Primer für NT3
<400> 5
   cggagcataa gagtcacc 18
<210> 6
   <211> 18
   <212> DNA
   <213> Artificial Sequence
<220>
   <223> Reverse Primer für NT3
<400> 6
   cctggcttcc ttacatcg 18
<210> 7
   <211> 20
   <212> DNA
   <213> Artificial Sequence
<220>
   <223> Forward Primer für Id2
<400> 7
   catcctgtcc ttgcaggctt 20
<210> 8
   <211> 23
   <212> DNA
   <213> Artificial Sequence
<220>
   <223> Reverse Primer für Id2
<400> 8
   ccattcaact tgtcctcctt gtg 23
<210> 9
   <211> 20
   <212> DNA
   <213> Artificial Sequence
<220>
   <223> Forward Primer für BTG2
<400> 9
   caggaggcac tcacagagca 20
<210> 10
   <211> 21
   <212> DNA
   <213> Artificial Sequence
<220>
   <223> Reverse Primer für BTG2
<400> 10
   aatgcggtag gacacctcat a 21
<210> 11
   <211> 17
   <212> DNA
   <213> Artificial Sequence
<220>
   <223> Forward Primer für Cbln
<400> 11
   caagtgcctg gtggtgt 17
<210> 12
   <211> 25
   <212> DNA
   <213> Artificial Sequence
<220>
   <223> Reverse Primer für Cbln
<400> 12
   gttcactagt acctggtcga agtag 25
<210> 13
   <211> 23
   <212> DNA
   <213> Artificial Sequence
<220>
   <223> Forward Primer für DRD2S
<400> 13
   ggactcaata acgcagacca gaa 23
<210> 14
   <211> 18
   <212> DNA
   <213> Artificial Sequence
<220>
   <223> Reverse Primer für DRD2S
<400> 14
   cgggcagcct cctttagt 18
<210> 15
   <211> 20
   <212> DNA
   <213> Artificial Sequence
<220>
   <223> Forward Primer für DKK1
<400> 15
   cattgacaac taccagccgt 20
<210> 16
   <211> 24
   <212> DNA
   <213> Artificial Sequence
<220>
   <223> Reverse Primer für DKK1
<400> 16
   atcagaagac acacatattc catt 24
<210> 17
   <211> 20
   <212> DNA
   <213> Artificial Sequence
<220>
   <223> Forward Primer für Bax
<400> 17
   gatgattgcc gccgtggaca 20
<210> 18
   <211> 21
   <212> DNA
   <213> Artificial Sequence
<220>
   <223> Reverse Primer für Bax
<400> 18
   caccttggtg cacagggcct t 21
<210> 19
   <211> 18
   <212> DNA
   <213> Artificial Sequence
<220>
   <223> Forward Primer für Bcl2
<400> 19
   gtgtggagag cgtcaacc 18
<210> 20
   <211> 19
   <212> DNA
   <213> Artificial Sequence
<220>
   <223> Reverse Primer für Bcl2
<400> 20
   cttcagagac agccaggag 19
<210> 21
   <211> 24
   <212> DNA
   <213> Artificial Sequence
<220>
   <223> Forward Primer für B2M (Referenzgen)
<400> 21
   actggtcttt ctatctcttg tact 24
<210> 22
   <211> 20
   <212> DNA
   <213> Artificial Sequence
<220>
   <223> Reverse Primer für B2M (Referenzgen)
<400> 22
   cttcaaacct ccatgatgct 20

## Beschreibung

Die vorliegende Erfindung betrifft Fluor-9-methyl-β-carboline, deren Herstellung sowie deren medizinische Verwendung und pharmazeutische Zusammensetzungen enthaltend Fluor-9-methyl-β-carboline.

Die erfindungsgemäßen Verbindungen dienen zur Behandlung von akuten und chronischen Ohrenerkrankungen und Hörschäden, Schwindel und Gleichgewichtsstörungen insbesondere von Hörsturz, Knalltrauma, Explosionstrauma, Tinnitus, Innenohrschwerhörigkeit durch chronische Lärmexposition, Altersschwerhörigkeit, Trauma während der Implantation von Innenohrprothesen (Insertionstrauma), Schwindel aufgrund von Erkrankungen im Innenohrbereich, Schwindel im Zusammenhang und/oder als Symptom der Menière'schen Erkrankung, Gleichgewichtsstörungen im Zusammenhang und/oder als Symptom der Menière'schen Erkrankung, und Hörschäden aufgrund von Antibiotika und Zytostatika.

Nach Erhebungen der Weltgesundheitsorganisation (WHO) leiden ca. 250 Millionen Menschen weltweit an leichten oder schweren Hörstörungen. In den USA sind 30 bis 40 Millionen Menschen von Hörschädigungen und von Hörverlusten betroffen. Allein hier betragen die Kosten der Behandlungen ca. 50 Milliarden USD jährlich. Der deutsche Schwerhörigenbund berichtete im Jahr 2007, dass ca. 19 % der deutschen Bevölkerung über 14 Jahre an Hörstörungen leiden.

Mit zunehmendem Alter nimmt der Prozentsatz an Hörgeschädigten zu. Bereits jetzt stellen die Hörstörungen bei über 65-jährigen Menschen die vierthäufigste chronische körperliche Beeinträchtigung, nach Erkrankungen der Knochen und Gelenke, Bluthochdruck und Herzkrankheiten, dar. Unter den 60 bis 69-jährigen sind 37 %, unter den 70-jährigen und älteren über 54 % der Bevölkerung von Hörschädigungen betroffen. In der Bundesrepublik Deutschland leiden ca. 12-15 Millionen Patienten an Innenohrschwerhörigkeit und ca. 2,9 Millionen Patienten an Tinnitus.

Der Begriff "Tinnitus aurium" oder kurz Tinnitus bezeichnet ein Symptom oder auch Syndrom, bei dem der Betroffene anhaltende oder wiederkehrende Geräusche wahrnimmt, wobei zwei Formen des Tinnitus unterschieden werden können. Beim relativ seltenen "objektiven Tinnitus" sind die vom Patienten wahrgenommenen Geräusche auch von außen wahrzunehmen oder zumindest zu messen. Sie beruhen in der Regel auf einer körpereigenen Schallquelle wie z.B. auf einem Blutgefäß, das nah am Innenohr vorbeiläuft. Beim deutlich häufigeren "subjektiven Tinnitus" beruhen die akustischen Phänomene hingegen nicht auf einer äußeren für andere Personen wahrnehmbaren Quelle. Damit ist der "subjektive Tinnitus" eine akustische Wahrnehmung, die unabhängig von einem Schall, der auf das Ohr wirkt, vom Patienten wahrgenommen wird. Die Ursachen für diese Wahrnehmung können vielfältig sein und unter anderem auf eine Störung der Hörfunktion des Innenohrs zurückzuführen sein. Man sollte jedoch den Tinnitus deutlich von akustischen Halluzinationen abgrenzen. Die Art der scheinbaren Geräusche, welche der Tinnitus-Patient wahrnimmt, ist sehr vielfältig. Man fasst unter anderem folgende akustische Eindrücke unter dem Begriff Tinnitus zusammen:
- Brumm- oder Pfeiftöne
- Zischen
- Rauschen
- Knacken oder Klopfen

Das Geräusch kann in seiner Intensität gleichbleibend sein, es kann jedoch auch einen rhythmisch-pulsierenden Charakter haben.

Das Ohr ist folgendermaßen aufgebaut. Von der Ohrmuschel aus führt der Gehörgang in das Innere des Ohrs. Der Gehörgang endet am Trommelfell. Diesen Teil des Ohrs bezeichnet man als Äußeres Ohr. Hinter dem Äußeren Ohr liegt ein Raum, das Mittelohr, auch Tympanon genannt, welches mit der Eustachischen Röhre und damit auch mit dem Nasopharynx verbunden ist. Das Mittelohr wird auf seiner einen Seite vom Trommelfell und auf der gegenüberliegenden Seite durch das Ovale Fenster und durch das Runde Fenster abgeschlossen. Hinter den beiden Fenstern schließt sich der als Innenohr bezeichnete Raum an. Das Innenohr beherbergt mehrere Organe wie das Gleichgewichtsorgan (Vestibularapparat) und die so genannte Schnecke (Cochlea) für die akustische Wahrnehmung. Diese wiederum enthält das Cortische Organ als Träger der Sensorzellen (Haarzellen) im Innenohr.

Die Membran des Runden Fensters bildet eine biologische Barriere zum Innenohrraum und stellt das größte Hindernis für die lokale Therapie von Schädigungen und Erkrankungen des Innenohres dar. Der verabreichte Wirkstoff muss diese Membran überwinden, um in den Innenohrraum zu gelangen. Der Wirkstoff kann aber nicht mechanisch-apparativ durch die Membran appliziert werden, da sonst die Membran durch diese Manipulation geschädigt wird. Er kann jedoch operativ (z.B. Injektion durch das Trommelfell) an der Rundfenstermembran lokal appliziert werden und dann die Rundfenstermembran penetrieren. So gelangt der Wirkstoff in den mit Perilymphe gefüllten Innenohrraum, der durch die miteinander verbundenen Kompartimente der Cochlea, nämlich der Scala tympani und der Scala vestibuli, gebildet wird. In einem durch Membranen abgetrennten Kompartiment zwischen der Scala tympani und der Scala vestibuli, der als Scala media (auch Ductus cochlearis) bezeichnet wird, befinden sich von Endolymphe umgeben die Sinneszellen des Hörorgans (innere und äußere Haarzellen). Diese werden zusammen mit den umliegenden Stützzellen als Cortisches Organ bezeichnet. Die Haarzellen sind bei Innenohrschwerhörigkeit jeglicher Ursache (Alter, Medikamente wie beispielsweise bestimmte Antibiotika und Zytostatika) sowie bei Tinnitus primär geschädigt. Über die Membranen, die die Scala media vom perilymphatischen Raum abtrennen, können Wirkstoffe auch in die Endolymphe und so oder direkt zu den Haarzellen gelangen. Da sowohl die perilymphatischen als auch die endolymphatischen Kompartimente der Cochlea mit denen des Gleichgewichtsorgans in Verbindung stehen, können Wirkstoffe von der Cochlea auch an die Sinneszellen des Vestibularapparates gelangen.

Die deutsche Patentanmeldung (DE 10 2007 009264 A1) offenbart 9-Alkyl-β-carboline, die aufgrund ihrer neuroprotektiven Wirkung zur Therapie und/oder Prophylaxe von Bewegungsstörungen und/oder neurologischen Krankheiten wie z.B. Alzheimer oder Parkinson angewendet werden können.

Von Hamann et al. (J. Hamann et al.; Neurochem Internat. 2008, 52, 688-700) konnte eine neuroprotektive Wirkung des β-Carbolins 9-Methyl-β-carbolin an primären Zellkulturen mesencephaler Neurone gezeigt werden. Die dort gezeigte erhöhte Überlebensrate der Neurone durch 9-Methyl-β-carbolin war jedoch nicht auf eine erhöhte Proliferation zurückzuführen. Im Gegenteil, die Applikation von 9-Methyl-β-carbolin führte zu einer Hemmung der Proliferation und zu einer gesteigerten Differenzierung der Neurone. Diese Wirkung von 9-Methyl-β-carbolin konnte an humanen Neuroblastom-Zellen (SH-SY5Y) bestätigt werden. Einhergehend mit der geförderten Differenzierung konnte eine gesteigerte Expression von neurotrophen Faktoren wie sonic hedgehog, Wnt1 und Wnt5a nachgewiesen werden. Zudem zeigte sich in der primären Zellkultur mesencephaler Neurone eine 9-Methyl-β-carbolin-induzierte Erhöhung der Zahl an dopaminergen Neuronen. Daher wird ein therapeutischer Nutzen von 9-Methyl-β-carbolin bei der Behandlung von Parkinson in Aussicht gestellt.

Die US-Patentanmeldung (US 2004/038970 A1) offenbart die Verwendung von β-Carbolinen als Wirkstoff zur Behandlung einer Vielzahl medizinischer Indikationen, einschließlich Tinnitus, wobei jedoch eine strukturell stark von 9-Alkyl-β-carbolinen und somit auch von 6- und/oder 7-Fluor-9-methyl-β-carbolinen abweichende Gruppe an β-Carbolinen genannt wird.

Die internationale Patentanmeldung (WO 2011/079841) offenbart β-Carboline, insbesondere 9-Alkyl-β-carboline und deren Verwendung zur Behandlung von Erkrankungen bzw. Schädigungen des Innenohrs. Es konnte gezeigt werden, dass die Applikation von 9-Alkyl-β-carbolinen die neuronale Differenzierung von humanen Neuroblastom-Zellen (SH-SY5Y) sowie die Expression von neurotrophen Faktoren mit neuroprotektiven Eigenschaften fördert. Dies konnte in Zellkulturen von Cochlea-Präparationen der Ratte bestätigt werden. Zusätzlich konnte am Meerschweinchen gezeigt werden, dass 9-Methyl-β-carbolin nach lokaler Applikation am Runden Fenster des Innenohrs in den perilymphatischen Raum des Innenohrs diffundieren kann und zu einer Erhöhung der elektrophysiologischen Aktivität der Haarsinneszellen des Cortischen Organs führt.

Der vorliegenden Erfindung liegt die Aufgabe zugrunde, einen Wirkstoff sowie pharmazeutische Formulierungen bereitzustellen, welche zur Prophylaxe und Behandlung von Hörschäden, Altersschwerhörigkeit (Presbyakusis), Schwindel und Gleichgewichtsstörungen sowie zur Verbesserung von Lernen und Gedächtnis eingesetzt werden können und einen Vorteil zum Stand der Technik aufweisen.

Diese Aufgabe wird durch die technische Lehre der unabhängigen Ansprüche gelöst. Weitere vorteilhafte Ausgestaltungen, Aspekte und Details der Erfindung ergeben sich aus den abhängigen Ansprüchen, der Beschreibung und den Beispielen.

### Fluor-9-methyl-β-carboline

Überraschend hat sich gezeigt, dass die erfindungsgemäßen Fluor-9-methyl-β-carboline der allgemeinen Formel (I) eine im Vergleich zu Verbindungen aus dem Stand der Technik (z.B. 9-Methy-β-carbolin) eine gesteigerte Wirksamkeit bei der Behandlung von Innenohrerkrankungen aufweisen. Sie bieten die Möglichkeit bei zugleich verringerter Toxizität in geringeren Dosen eingesetzt zu werden, was eine deutliche therapeutische Verbesserung im Vergleich zum Stand der Technik darstellt. Zudem hat sich überraschenderweise gezeigt, dass sich die erfindungsgemäßen 6-und/oder 7-Fluor-9-methyl-β-carboline durch besondere Eigenschaften vom Stand der Technik abheben, die eine therapeutische Verwendung begünstigen. So konnte für die erfindungsgemäßen 6- und/oder 7-Fluor-9-methyl-β-carboline eine höhere Wirksamkeit im Vergleich zu 9-Methyl-β-carbolin bei der Förderung der neuronalen Differenzierung in humanen Neuroblastom-Zellen nachgewiesen werden. Zudem zeigen 6- und/oder 7-Fluor-9-methyl-β-carbolin im Vergleich zu 9-Methyl-β-carbolin eine deutlich erhöhte Lipophilie, was mit einer erhöhten Membranpermeabilität einhergeht. Diese hat weitreichende Konsequenzen für die therapeutische Anwendung der erfindungsgemäßen Verbindung zur Behandlung von Erkrankungen bzw. Schädigungen des Innenohres oder zur Verbesserung des Gedächtnisses und zur Steigerung der Lernfähigkeit. Wie bereits beschrieben bilden biologische Membranen wie das Trommelfell, die Membran des Runden Fensters und die Membranen der Scala media (Membrana tectoriana und Reißner Membran) ein natürliches Hindernis für einen Wirkstoff auf dem Weg zu den Haarzellen des Innenohres. Dies gilt insbesondere bei der topischen Applikation eines Wirkstoffes auf dem Trommelfell oder dem Runden Fenster. Durch eine erhöhte Membranpermeabilität wird daher die Verfügbarkeit des Wirkstoffes an den Zielzellen erhöht. Neben der Membranpermeabilität spielt auch der Abbau eines Wirkstoffs im Körper eine entscheidende Rolle für dessen Wirksamkeit, hier insbesondere bei systemischer Verabreichung, und auch für eventuelle Nebenwirkungen. Während das 9-Methyl-β-carbolin über eine Hydroxylierung an Position 6 mit anschließender Konjugationsreaktion abgebaut wird, ist dieser Metabolisierungsweg beim 6-Fluor-9-methyl-β-carbolin durch die Fluoridgruppe(n) blockiert. Hierdurch konnte nicht nur die Halbwertszeit des 6-Fluor-9-methyl-β-carbolins erhöht, sondern zudem das Risiko für die Bildung potentiell toxischer Abbauprodukte verringert werden. Aufgrund der erhöhten Wirksamkeit des 6- und/oder 7-Fluor-9-methyl-β-carbolins erscheint eine im Vergleich zu anderen 9-Alkyl-β-carbolinen geringere Dosierung bei der Therapie möglich. Dies ist gerade für eine Langzeittherapie bei chronischen Erkrankungen von enormer Bedeutung, da hierdurch die Belastung des Körpers und zudem unerwünschte Nebenwirkungen verringert werden können. Es handelt sich folglich bei der vorliegenden Anmeldung um eine Auswahlerfindung.

Unter dem Begriff "6- und/oder 7-Fluor-9-methyl-β-carbolin" sollen bei einer "oder"-Verknüpfung entweder die Verbindung 6-Fluor-9-methyl-β-carbolin oder die Verbindung 7-Fluor-9-methyl-β-carbolin gemeint sein und bei einer "und"-Verknüpfung die Verbindungen 6-Fluor-9-methyl-β-carbolin und 7-Fluor-9-methyl-β-carbolin.

Die vorliegende Erfindung betrifft Verbindungen der allgemeinen Formel (I) bzw. Fluor-9-methyl-β-carboline der allgemeinen Formel (I): 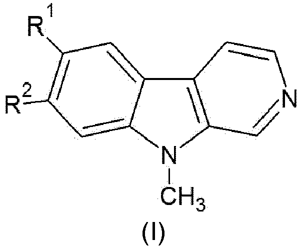
wobei R¹ = -F und R² = -H oder -F ist, oder
wobei R¹ = -H und R² = -F ist;
sowie pharmakologisch verträgliche Salze, Solvate und Hydrate sowie Komplexverbindungen der vorgenannten Verbindungen.

Die vorliegende Erfindung betrifft ebenfalls Verbindungen der allgemeinen Formel (I) bzw. Fluor-9-methyl-β-carboline der allgemeinen Formel (I): 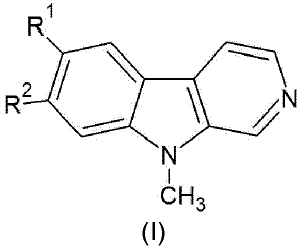
wobei R¹ und R² unabhängig voneinander -F oder -H sind, und
wobei zumindest einer der beiden Reste R¹ oder R² = -F ist;
sowie pharmakologisch verträgliche Salze, Solvate und Hydrate sowie Komplexverbindungen der vorgenannten Verbindungen.

Die vorliegende Erfindung betrifft ebenfalls Verbindungen der allgemeinen Formel (I) bzw. Fluor-9-methyl-β-carboline der allgemeinen Formel (I): 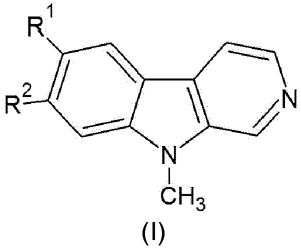
wobei R¹ und R² unabhängig voneinander -F oder -H sind, und
wobei zumindest einer der beiden Reste R¹ oder R² nicht -H ist;
sowie pharmakologisch verträgliche Salze, Solvate und Hydrate sowie Prodrugs und Komplexverbindungen der vorgenannten Verbindungen.
Bevorzugt ist, wenn R¹ = -F und R² = -H oder wenn R¹ = -H und R² = -F ist.

Der hierin verwendete Ausdruck Prodrug ist definiert als eine pharmakologische Substanz, die in einer inaktiven oder weniger wirksamen Form verabreicht wird. Nach Gabe wird sie dann im Körper des Patienten in ihre aktive, wirksame Form, z.B. ein Fluor-9-methyl-β-carbolin der allgemeinen Formel (I), umgewandelt.

Als mögliche Säuren, welche ein Säureadditionssalz mit der Verbindung der vorliegenden Erfindung bilden, können die folgenden genannt werden: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-Mandelsäure, Hydrogenbenzolsulfonsäure, Pikrinsäure (2,4,6-Trinitrophenol), Adipinsäure, d-o-Tolylweinsäure, Aminosäuren wie Methionin, Tryptophan, Arginin und insbesondere saure Aminosäuren wie Glutaminsäure oder Asparaginsäure. Zudem sind auch Betain-Formen möglich.

Ein weiterer Aspekt der vorliegenden Erfindung betrifft ein Verfahren zur Herstellung der erfindungsgemäßen Verbindung der allgemeinen Formel (I) (bzw. eines Fluor-9-methyl-β-carbolins der allgemeinen Formel (I)), das die folgenden Schritte umfasst:
a) Reaktion der Ausgangsverbindung der allgemeinen Formel (II) (bzw. eines Fluor-1-methyltryptamin-hydrochlorids der allgemeinen Formel (II)) unter Zugabe von Glyoxalsäurehydrat der Formel (III) und einer Base zu einer Verbindung der allgemeinen Formel (IV) 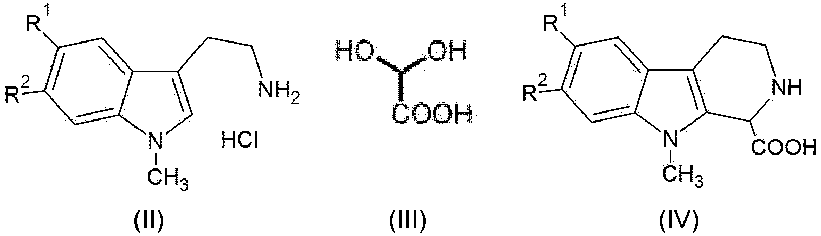
b) Decarboxylierung der Verbindung der allgemeinen Formel (IV) unter Zugabe einer Säure und unter Erhitzen zu einer Verbindung der allgemeinen Formel (V) (bzw. zu einem Fluor-9-methyl-2,3,4,9-tetrahydro-β-carbolin der allgemeinen Formel (V)) 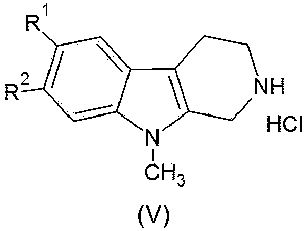
c) Aromatisierung der Verbindung der allgemeinen Formel (V) (bzw. des Fluor-9-methyl-2,3,4,9-tetrahydro-β-carbolins der allgemeinen Formel (V)) zu einer Verbindung der allgemeinen Formel (I) (bzw. zu einem Fluor-9-methyl-β-carbolin der allgemeinen Formel (I)) unter Zugabe eines Katalysators;
wobei
R¹ = -F und R² = -H oder -F ist, oder
R¹ = -H und R² = -F ist.

Es versteht sich von selbst, dass die Repräsentation der Reste R¹ und R², wie oben aufgeführt, für ein bestimmtes Verfahren zur Herstellung einer erfindungsgemäßen Verbindung nicht nur für das Ausgangsprodukt der jeweiligen Synthese, sondern auch für die daraus gewonnenen Zwischenprodukte sowie für das letztliche Endprodukt der Synthese gelten.

Ein weiterer Aspekt der vorliegenden Erfindung betrifft daher ein Verfahren zur Herstellung der erfindungsgemäßen Verbindung der allgemeinen Formel (I) (bzw. eines Fluor-9-methyl-β-carbolins der allgemeinen Formel (I)), das die folgenden Schritte umfasst:
a) Reaktion der Ausgangsverbindung der allgemeinen Formel (II) (bzw. eines Fluor-1-methyltryptamin-hydrochlorids der allgemeinen Formel (II)) unter Zugabe von Glyoxalsäurehydrat der Formel (III) und einer Base zu einer Verbindung der allgemeinen Formel (IV) 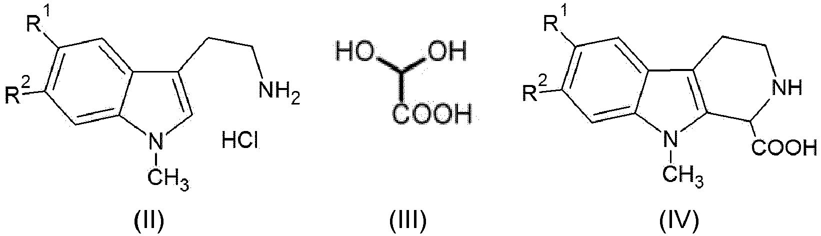
b) Decarboxylierung der Verbindung der allgemeinen Formel (IV) unter Zugabe einer Säure und unter Erhitzen zu einer Verbindung der allgemeinen Formel (V) (bzw. zu einem Fluor-9-methyl-2,3,4,9-tetrahydro-β-carbolin der allgemeinen Formel (V)) 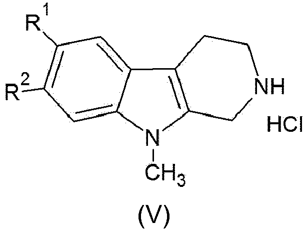
c) Aromatisierung der Verbindung der allgemeinen Formel (V) (bzw. des Fluor-9-methyl-2,3,4,9-tetrahydro-β-carbolins der allgemeinen Formel (V)) zu einer Verbindung der allgemeinen Formel (I) (bzw. zu einem Fluor-9-methyl-β-carbolin der allgemeinen Formel (I)) unter Zugabe eines Katalysators;

wobei R¹ = -F und R² = -H oder -F ist und dies gleichermaßen für die Verbindungen der allgemeinen Formel (I), (II), (IV) und (V) gilt,
oder wobei R¹ = -H und R² = -F ist und dies gleichermaßen für die Verbindungen der allgemeinen Formel (I), (II), (IV) und (V) gilt.

Die Fluor-1-methyltryptaminhydrochloride als Ausgangsprodukte sind kommerziell erhältlich (siehe auch unten). In einer bevorzugten Ausführungsform der Erfindung wird durch Zugabe der Base in Schritt a) ein pH-Wert von zwischen 2 und 6, bevorzugt zwischen 3 und 5 und besonders bevorzugt ein pH-Wert von 4 erreicht. In einer weiteren bevorzugten Ausführungsform wird die Kristallisation der Verbindung IV in einem Schritt a)' durch eine Inkubation in Eiswasser gefördert.

In einer weiteren bevorzugten Ausführungsform erfolgt die Reaktion in Schritt b) unter Inertgas, besonders bevorzugt unter N₂. Des Weiteren wird bevorzugt, die Kristallisation des Reaktionsproduktes aus Schritt b) durch einen Schritt b)' Inkubation bei Kälte, besonders bevorzugt zwischen 0°C und 5°C, über mehrere Stunden zu fördern. In einer weiteren bevorzugten Ausführungsform wird zur Aufreinigung des Reaktionsproduktes aus Schritt b) nach der Kristallisation eine Umkristallisation aus Methanol durchgeführt. Des Weiteren wird bevorzugt, das Fluor-9-methyl-2,3,4,9-tetrahydro-β-carbolin (bzw. die Verbindung der allgemeinen Formel (V)) in Schritt c) zunächst in Wasser zu lösen und durch Zugabe einer Base, z.B. Natriumhydroxid oder Natriumcarbonat, auf einen basischen pH-Wert einzustellen, besonders bevorzugt einen pH-Wert zwischen pH 11 und pH 14, besonders bevorzugt pH 13.

In einer bevorzugten Ausführungsform, ist als Katalysator für die oxidative Aromatisierung in Schritt c) unter anderem Pd / C, Pt / C, PdCl₂, Pd / Sepiolith, Pd / SiO₂ und Pd / AlO₄ möglich. Dem Fachmann sind zudem weitere Zusätze bekannt, die eine oxidative Aromatisierung ermöglichen. Bevorzugt wird jedoch Pd / C (*Palladium on carbon*, Palladium auf Aktivkohle) als Katalysator eingesetzt.

Bei der Zugabe einer Base in Schritt a) und Schritt c) kann es sich unabhängig voneinander um anorganische oder organische Basen wie z.B. NaOH, KOH, NH₄OH, Tetraalkylammonium Hydroxid, Lysin oder Arginin handeln. Dem Fachmann ist bekannt, welche Basen sich hierbei zur Durchführung der Reaktion eignen. Bevorzugt sind jedoch KOH bzw. NaOH.

Bei der Zugabe einer Säure in Schritt b), kann es sich um anorganische und organische Säuren wie z.B. Salzsäure, Bromwasserstoffsäure, Schwefelsäure, Phosphorsäure, Essigsäure, Zitronensäure, Oxalsäure, Äpfelsäure, Salicylsäure, p-Aminosalicylsäure, Malonsäure, Fumarsäure, Bernsteinsäure, Ascorbinsäure, Maleinsäure, Sulfonsäure, Phosphonsäure, Perchlorsäure, Salpetersäure, Ameisensäure, Propionsäure, Gluconsäure, Milchsäure, Weinsäure, Hydroxymaleinsäure, Brenztraubensäure, Phenylessigsäure, Benzoesäure, p-Aminobenzoesäure, p-Hydroxybenzoesäure, Methansulfonsäure, Ethansulfonsäure, salpetrige Säure, Hydroxyethansulfonsäure, Ethylensulfonsäure, p-Toluolsulfonsäure, Naphthylsulfonsäure, Sulfanilsäure, Camphersulfonsäure, Chinasäure, Mandelsäure, o-Methylmandelsäure, Hydrogenbenzolsulfonsäure, Pikrinsäure, Adipinsäure, d-o-Tolylweinsäure, Tartronsäure, α-Toluylsäure, (o-, m-, p-) Toluylsäure, Naphthylaminsulfonsäure handeln. Dem Fachmann ist bekannt, welche Säure sich hierbei zur Durchführung der Reaktion eignet. Bevorzugt ist jedoch Salzsäure.

Gemäß weiteren Ausführungsformen ist die vorliegende Erfindung gerichtet auf ein Fluor-9-methyl-β-carbolin der allgemeinen Formel (I) 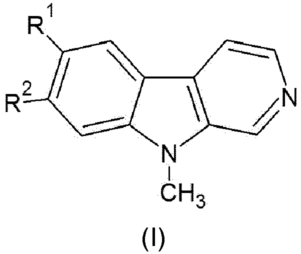 wobei R¹ = -F und R² = -F ist, noch mehr bevorzugt wobei R¹ = -H und R² = -F ist, und am meisten bevorzugt wobei R¹ = -F und R² = -H ist.

### 6-Fluor-9-methyl-β-carbolin (6F9MβC)

Gemäß einer besonders bevorzugten Ausführungsform betriff die vorliegende Erfindung das β-Carbolin 6-Fluor-9-methyl-β-carbolin, dessen Herstellung sowie dessen medizinische Verwendung und pharmazeutische Zusammensetzungen enthaltend 6-Fluor-9-methyl-β-carbolin.

Die Verbindung 6-Fluor-9-methyl-β-carbolin wird hierin zum Teil auch mit "6F9MβC" oder "AC102" abgekürzt. Das 9-Methyl-β-carbolin aus dem Stand der Technik hingegen wird hierin zum Teil auch mit "9MβC" oder "AC002" abgekürzt.

Die erfindungsgemäße Verbindung 6-Fluor-9-methyl-β-carbolin ist im Stand der Technik nicht in individualisierter, d.h. konkreter Form offenbart. Zudem hat sich überraschenderweise gezeigt, dass sich das erfindungsgemäße 6-Fluor-9-methyl-β-carbolin durch besondere Eigenschaften vom Stand der Technik abhebt, die eine therapeutische Verwendung begünstigen. So konnte für das erfindungsgemäße 6-Fluor-9-methyl-β-carbolin eine höhere Wirksamkeit im Vergleich zu 9-Methyl-β-carbolin bei der Förderung der neuronalen Differenzierung in humanen Neuroblastom-Zellen nachgewiesen werden (siehe Beispiel 2). Zudem zeigt 6-Fluor-9-methyl-β-carbolin im Vergleich zu 9-Methyl-β-carbolin eine deutlich erhöhte Lipophilie, was mit einer erhöhten Membranpermeabilität einhergeht. Diese hat weitreichende Konsequenzen für die therapeutische Anwendung der erfindungsgemäßen Verbindung zur Behandlung von Erkrankungen bzw. Schädigungen des Innenohres. Wie bereits beschrieben bilden biologische Membranen wie das Trommelfell, die Membran des Runden Fensters und die Membranen der Scala media (Membrana tectoriana und Reißner Membran) ein natürliches Hindernis für einen Wirkstoff auf dem Weg zu den Haarzellen des Innenohres. Dies gilt insbesondere bei der topischen Applikation eines Wirkstoffes auf dem Trommelfell oder dem Runden Fenster. Durch eine erhöhte Membranpermeabilität wird daher die Verfügbarkeit des Wirkstoffes an den Zielzellen erhöht. Neben der Membranpermeabilität spielt auch der Abbau eines Wirkstoffs im Körper eine entscheidende Rolle für dessen Wirksamkeit, hier insbesondere bei systemischer Verabreichung, und auch für eventuelle Nebenwirkungen. Während das 9-Methyl-β-carbolin über eine Hydroxylierung an Position 6 mit anschließender Konjugationsreaktion abgebaut wird, ist dieser Metabolisierungsweg beim 6-Fluor-9-methyl-β-carbolin durch die Fluoridgruppe blockiert. Hierdurch konnte nicht nur die Halbwertszeit des 6-Fluor-9-methyl-β-carbolins erhöht, sondern zudem das Risiko für die Bildung potentiell toxischer Abbauprodukte verringert werden. Aufgrund der erhöhten Wirksamkeit des 6-Fluor-9-methyl-β-carbolins erscheint eine im Vergleich zu anderen 9-Alkyl-β-carbolinen geringere Dosierung bei der Therapie möglich. Dies ist gerade für eine Langzeittherapie bei chronischen Erkrankungen von enormer Bedeutung, da hierdurch die Belastung des Körpers und zudem unerwünschte Nebenwirkungen verringert werden können.

Die vorliegende Erfindung betrifft die Verbindung 6-Fluor-9-methyl-β-carbolin der Formel (la) 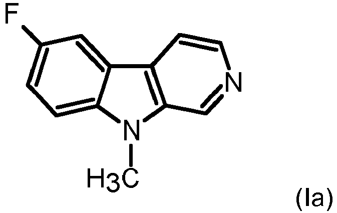 sowie pharmakologisch verträgliche Salze, Solvate und Hydrate sowie Komplexverbindungen der vorgenannten Verbindung.

Der hierin verwendete Ausdruck Prodrug ist definiert als eine pharmakologische Substanz, die in einer inaktiven oder weniger wirksamen Form verabreicht wird. Nach Gabe wird sie dann im Körper des Patienten in ihre aktive, wirksame Form, im obigen Fall das 6-Fluor-9-methyl-β-carbolin, umgewandelt.

Als mögliche Säuren, welche ein Säureadditionssalz mit der erfindungsgemäßen Verbindung 6-Fluor-9-methyl-β-carbolin bilden, können die folgenden genannt werden: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-Mandelsäure, Hydrogenbenzolsulfonsäure, Pikrinsäure (2,4,6-Trinitrophenol), Adipinsäure, d-o-Tolylweinsäure, Aminosäuren wie Methionin, Tryptophan, Arginin und insbesondere saure Aminosäuren wie Glutaminsäure oder Asparaginsäure. Zudem sind auch Betain-Formen möglich.

Ein weiterer Aspekt der vorliegenden Erfindung betrifft ein Verfahren zur Herstellung der erfindungsgemäßen Verbindung 6-Fluor-9-methyl-β-carbolin, das die folgenden Schritte umfasst:
a) Reaktion der Ausgangsverbindung 5-Fluor-1-methyltryptamin-hydrochlorid der Formel (IIa) unter Zugabe von Glyoxalsäurehydrat der Formel (III) und einer Base zur Verbindung der Formel (IVa) 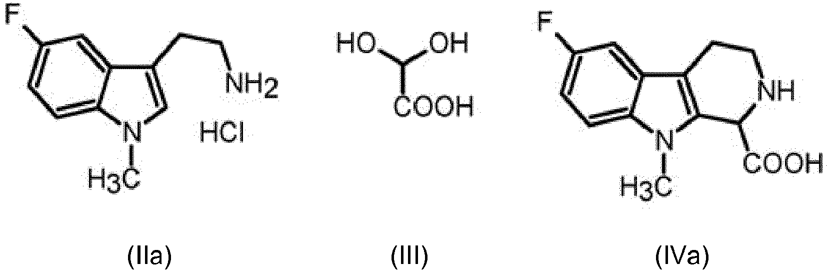
b) Decarboxylierung der Verbindung der Formel (IVa) unter Zugabe einer Säure und unter Erhitzen zu 6-Fluor-9-methyl-2,3,4,9-tetrahydro-β-carbolin der Formel (Va) 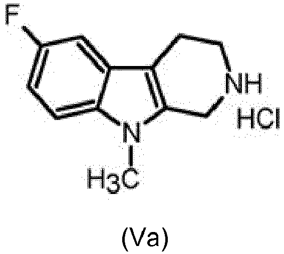
c) Aromatisierung von 6-Fluor-9-methyl-2,3,4,9-tetrahydro-β-carbolin (Va) zu 6-Fluor-9-methyl-β-carbolin (la) unter Zugabe eines Katalysators.

Das Ausgangsprodukt 5-Fluor-1-methyltryptaminhydrochlorid ist kommerziell erhältlich, z.B. über die AKos GmbH (Steinen). In einer bevorzugten Ausführungsform der Erfindung wird durch Zugabe der Base in Schritt a) ein pH-Wert von zwischen 2 und 6, bevorzugt zwischen 3 und 5 und besonders bevorzugt ein pH-Wert von 4 erreicht. In einer weiteren bevorzugten Ausführungsform wird die Kristallisation der Verbindung IVa in einem Schritt a)' durch eine Inkubation in Eiswasser gefördert.

In einer weiteren bevorzugten Ausführungsform erfolgt die Reaktion in Schritt b) unter Inertgas, besonders bevorzugt unter N₂. Des Weiteren wird bevorzugt, die Kristallisation des Reaktionsproduktes aus Schritt b) durch einen Schritt b)' Inkubation bei Kälte, besonders bevorzugt zwischen 0°C und 5°C, über mehrere Stunden zu fördern. In einer weiteren bevorzugten Ausführungsform wird zur Aufreinigung des Reaktionsproduktes aus Schritt b) nach der Kristallisation eine Umkristallisation aus Methanol durchgeführt. Des Weiteren wird bevorzugt, das 6-Fluor-9-methyl-2,3,4,9-tetrahydro-β-carbolin in Schritt c) zunächst in Wasser zu lösen und durch Zugabe einer Base, z.B. Natriumhydroxid oder Natriumcarbonat, auf einen basischen pH-Wert einzustellen, besonders bevorzugt einen pH-Wert zwischen pH 11 und pH 14, besonders bevorzugt pH 13.

In einer bevorzugten Ausführungsform, ist als Katalysator für die oxidative Aromatisierung in Schritt c) unter anderem Pd / C, Pt / C, PdCl₂, Pd / Sepiolith, Pd/SiO₂ und Pd/AlO₄ möglich. Dem Fachmann sind zudem weitere Zusätze bekannt, die eine oxidative Aromatisierung ermöglichen. Bevorzugt wird jedoch Pd / C (*Palladium on carbon*, Palladium auf Aktivkohle) als Katalysator eingesetzt.

Bei der Zugabe einer Base in Schritt a) und Schritt c) kann es sich unabhängig voneinander um anorganische oder organische Basen wie z.B. NaOH, KOH, NH₄OH, Tetraalkylammonium Hydroxid, Lysin oder Arginin handeln. Dem Fachmann ist bekannt, welche Basen sich hierbei zur Durchführung der Reaktion eignen. Bevorzugt sind jedoch KOH bzw. NaOH.

Bei der Zugabe einer Säure in Schritt b), kann es sich um anorganische und organische Säuren wie z.B. Salzsäure, Bromwasserstoffsäure, Schwefelsäure, Phosphorsäure, Essigsäure, Zitronensäure, Oxalsäure, Äpfelsäure, Salicylsäure, p-Aminosalicylsäure, Malonsäure, Fumarsäure, Bernsteinsäure, Ascorbinsäure, Maleinsäure, Sulfonsäure, Phosphonsäure, Perchlorsäure, Salpetersäure, Ameisensäure, Propionsäure, Gluconsäure, Milchsäure, Weinsäure, Hydroxymaleinsäure, Brenztraubensäure, Phenylessigsäure, Benzoesäure, p-Aminobenzoesäure, p-Hydroxybenzoesäure, Methansulfonsäure, Ethansulfonsäure, salpetrige Säure, Hydroxyethansulfonsäure, Ethylensulfonsäure, p-Toluolsulfonsäure, Naphthylsulfonsäure, Sulfanilsäure, Camphersulfonsäure, Chinasäure, Mandelsäure, o-Methylmandelsäure, Hydrogenbenzolsulfonsäure, Pikrinsäure, Adipinsäure, d-o-Tolylweinsäure, Tartronsäure, α-Toluylsäure, (o-, m-, p-) Toluylsäure, Naphthylaminsulfonsäure handeln. Dem Fachmann ist bekannt, welche Säure sich hierbei zur Durchführung der Reaktion eignet. Bevorzugt ist jedoch Salzsäure.

Es konnte überraschenderweise gezeigt werden, dass die erfindungsgemäße Verbindung 6-Fluor-9-methyl-β-carbolin (la) im Vergleich zu bereits bekannten und getesteten β-Carbolinen, wie 9-Methyl-β-Carbolin eine gesteigerte Wirksamkeit bei der Behandlung von Innenohrerkrankungen aufweist. Dies zeigte sich sowohl bei Untersuchungen zur Wirkstoff-induzierten Genexpression wichtiger zellulärer Faktoren (siehe Beispiel 4), die für die zelluläre Regeneration (vor allem im Innenohr) nach Traumata von Bedeutung sind, als auch in histologischen (siehe Beispiel 6) sowie physiologischen Untersuchungen (siehe Beispiel 7) zur Regeneration des auditorischen Systems nach einem erfolgten Schalltrauma. Zusätzlich zeigen mehrere Experimente eine deutliche erhöhte Lipophilie (siehe Beispiel 3) der erfindungsgemäßen Verbindung 6-Fluor-9-methyl-β-carbolin (la). Gegenüber der Vergleichssubstanz 9-Methyl-β-carbolin ergeben sich u.a. die folgenden Vorteile:
1. Die Membrangängigkeit ist erhöht.
2. Die Elimination ist verlangsamt (Eliminationshalbwertszeit von 6F9MβC ca 1,75-fach höher als für 9MβC).
3. Da von anderen β-Carbolinen bekannt ist, dass sie durch Hydroxylierung an Position 6 und eine anschließende Konjugationsreaktion abgebaut werden, ist durch eine Blockierung dieses Metabolisierungsweges durch die Fluoridgruppe an Position 6.
   - Die Bioverfügbarkeit nach oraler Applikation ist verbessert.
   - Das Risiko von toxischen Metaboliten oder potentiell allergenen Metaboliten minimiert.
4. Ein weiterer bekannter Metabolisierungsweg von β-Carbolinen, der zu toxischen di-methylierten Abbauprodukten führt, verläuft über die sequentielle N-Methylierung in vivo, wobei zunächst das Stickstoffatom an Position 2 und dann das Stickstoffatom an Position 9 methyliert wird. In aktuellen Experimenten konnte gezeigt werden, dass das 6F9MβC erfreulicherweise nicht zu den toxischen di-methylierten Produkten führt (siehe Beispiel 8), wodurch das Risiko von toxischen Metaboliten weiter minimiert ist.
5. gesteigerte Wirksamkeit bei der Wirkstoff-induzierten Genexpression wichtiger zellulärer Faktoren (siehe Beispiel 4), die für die zelluläre Regeneration (vor allem im Innenohr) nach Traumata von Bedeutung sind, als auch in histologischen (siehe Beispiel 6) sowie physiologischen Untersuchungen (siehe Beispiel 7) zur Regeneration des auditorischen Systems nach einem erfolgten Schalltrauma.

### 7-Fluor-9-methyl-β-carbolin (7F9MβC)

Gemäß einer weiteren Ausführungsform betrifft die vorliegende Erfindung das β-Carbolin 7-Fluor-9-methyl-β-carbolin, dessen Herstellung sowie dessen medizinische Verwendung und pharmazeutische Zusammensetzungen enthaltend 7-Fluor-9-methyl-β-carbolin.

Die Verbindung 7-Fluor-9-methyl-β-carbolin wird hierin zum Teil auch mit "7F9MβC" abgekürzt.

Die vorliegende Erfindung betrifft die Verbindung 7-Fluor-9-methyl-β-carbolin der Formel (Ib) 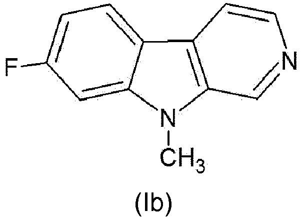 sowie pharmakologisch verträgliche Salze, Solvate und Hydrate sowie Prodrugs und Komplexverbindungen der vorgenannten Verbindung.

Der hierin verwendete Ausdruck Prodrug ist, wie bereits erläutert, definiert als eine pharmakologische Substanz, die in einer inaktiven oder weniger wirksamen Form verabreicht wird. Nach Gabe wird sie dann im Körper des Patienten in ihre aktive, wirksame Form, im obigen Fall das 7-Fluor-9-methyl-β-carbolin, umgewandelt.

Als mögliche Säuren, welche ein Säureadditionssalz mit der erfindungsgemäßen Verbindung 7-Fluor-9-methyl-β-carbolin bilden, können die folgenden genannt werden: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-Mandelsäure, Hydrogenbenzolsulfonsäure, Pikrinsäure (2,4,6-Trinitrophenol), Adipinsäure, d-o-Tolylweinsäure, Aminosäuren wie Methionin, Tryptophan, Arginin und insbesondere saure Aminosäuren wie Glutaminsäure oder Asparaginsäure. Zudem sind auch Betain-Formen möglich.

Ein weiterer Aspekt der vorliegenden Erfindung betrifft ein Verfahren zur Herstellung der erfindungsgemäßen Verbindung 7-Fluor-9-methyl-β-carbolin, das die folgenden Schritte umfasst:
a) Reaktion der Ausgangsverbindung 6-Fluor-1-methyltryptamin-hydrochlorid der Formel (IIb) unter Zugabe von Glyoxalsäurehydrat der Formel (III) und einer Base zur Verbindung der Formel (IVb) 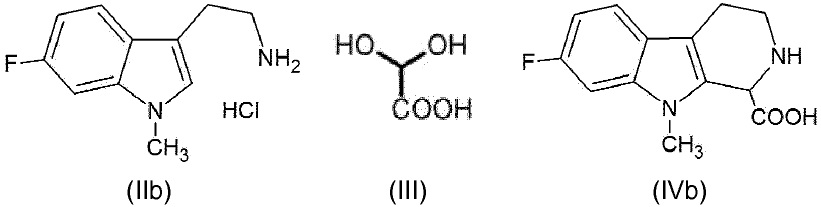
b) Decarboxylierung der Verbindung der Formel (IVb) unter Zugabe einer Säure und unter Erhitzen zu 7-Fluor-9-methyl-2,3,4,9-tetrahydro-β-carbolin der Formel (Vb) 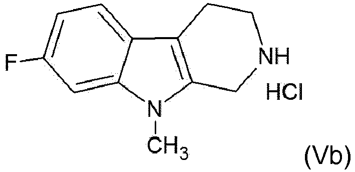
c) Aromatisierung von 7-Fluor-9-methyl-2,3,4,9-tetrahydro-β-carbolin (Vb) zu 7-Fluor-9-methyl-β-carbolin (Ib) unter Zugabe eines Katalysators.

Das Ausgangsprodukt 6-Fluor-1-methyltryptaminhydrochlorid ist kommerziell erhältlich, z.B. über die Otavachemicals Ltd. (Vaughan, Kanada). In einer bevorzugten Ausführungsform der Erfindung wird durch Zugabe der Base in Schritt a) ein pH-Wert von zwischen 2 und 6, bevorzugt zwischen 3 und 5 und besonders bevorzugt ein pH-Wert von 4 erreicht. In einer weiteren bevorzugten Ausführungsform wird die Kristallisation der Verbindung IVb in einem Schritt a)' durch eine Inkubation in Eiswasser gefördert.

In einer weiteren bevorzugten Ausführungsform erfolgt die Reaktion in Schritt b) unter Inertgas, besonders bevorzugt unter N₂. Des Weiteren wird bevorzugt, die Kristallisation des Reaktionsproduktes aus Schritt b) durch einen Schritt b)' Inkubation bei Kälte, besonders bevorzugt zwischen 0°C und 5°C, über mehrere Stunden zu fördern. In einer weiteren bevorzugten Ausführungsform wird zur Aufreinigung des Reaktionsproduktes aus Schritt b) nach der Kristallisation eine Umkristallisation aus Methanol durchgeführt. Des Weiteren wird bevorzugt, das 7-Fluor-9-methyl-2,3,4,9-tetrahydro-β-carbolin in Schritt c) zunächst in Wasser zu lösen und durch Zugabe einer Base, z.B. Natriumhydroxid oder Natriumcarbonat, auf einen basischen pH-Wert einzustellen, besonders bevorzugt einen pH-Wert zwischen pH 11 und pH 14, besonders bevorzugt pH 13.

In einer bevorzugten Ausführungsform, ist als Katalysator für die oxidative Aromatisierung in Schritt c) unter anderem Pd / C, Pt / C, PdCl₂, Pd / Sepiolith, Pd/SiO₂ und Pd/AlO₄ möglich. Dem Fachmann sind zudem weitere Zusätze bekannt, die eine oxidative Aromatisierung ermöglichen. Bevorzugt wird jedoch Pd / C (*Palladium on carbon*, Palladium auf Aktivkohle) als Katalysator eingesetzt.

Bei der Zugabe einer Base in Schritt a) und Schritt c) kann es sich unabhängig voneinander um anorganische oder organische Basen wie z.B. NaOH, KOH, NH₄OH, Tetraalkylammonium Hydroxid, Lysin oder Arginin handeln. Dem Fachmann ist bekannt, welche Basen sich hierbei zur Durchführung der Reaktion eignen. Bevorzugt sind jedoch KOH bzw. NaOH.

Bei der Zugabe einer Säure in Schritt b), kann es sich um anorganische und organische Säuren wie z.B. Salzsäure, Bromwasserstoffsäure, Schwefelsäure, Phosphorsäure, Essigsäure, Zitronensäure, Oxalsäure, Äpfelsäure, Salicylsäure, p-Aminosalicylsäure, Malonsäure, Fumarsäure, Bernsteinsäure, Ascorbinsäure, Maleinsäure, Sulfonsäure, Phosphonsäure, Perchlorsäure, Salpetersäure, Ameisensäure, Propionsäure, Gluconsäure, Milchsäure, Weinsäure, Hydroxymaleinsäure, Brenztraubensäure, Phenylessigsäure, Benzoesäure, p-Aminobenzoesäure, p-Hydroxybenzoesäure, Methansulfonsäure, Ethansulfonsäure, salpetrige Säure, Hydroxyethansulfonsäure, Ethylensulfonsäure, p-Toluolsulfonsäure, Naphthylsulfonsäure, Sulfanilsäure, Camphersulfonsäure, Chinasäure, Mandelsäure, o-Methylmandelsäure, Hydrogenbenzolsulfonsäure, Pikrinsäure, Adipinsäure, d-o-Tolylweinsäure, Tartronsäure, α-Toluylsäure, (o-, m-, p-) Toluylsäure, Naphthylaminsulfonsäure handeln. Dem Fachmann ist bekannt, welche Säure sich hierbei zur Durchführung der Reaktion eignet. Bevorzugt ist jedoch Salzsäure.

Es konnte überraschenderweise gezeigt werden, dass die erfindungsgemäße Verbindung 7-Fluor-9-methyl-β-carbolin (Ib) im Vergleich zu bereits bekannten und getesteten β-Carbolinen, wie 9-Methyl-β-Carbolin, eine gesteigerte Wirksamkeit bei der Behandlung von Innenohrerkrankungen aufweist. Anhand der aktuellen Untersuchungen zur Wirksamkeit des 7-Fluor-9-methyl-β-carbolins gegenüber der Vergleichssubstanz 9-Methyl-β-carbolin sich u.a. die folgenden Vorteile:
1. Die Membrangängigkeit ist erhöht.
2. Die Elimination ist verlangsamt (Eliminationshalbwertszeit von 7F9MβC ca 1,5-fach höher als für 9MβC).
3. gesteigerte Wirksamkeit bei der Wirkstoff-induzierten Genexpression wichtiger zellulärer Faktoren (siehe Beispiel 4 und 5), die für die zelluläre Regeneration (vor allem im Innenohr) nach Traumata von Bedeutung sind.

### Pharmazeutische Anwendungen & Formulierungen

Die erfindungsgemäßen Verbindungen sind besonders geeignet zur Behandlung von Erkrankungen bzw. Schädigungen des Innenohrs, insbesondere der neuronalen und sensorischen Strukturen des Innenohres. Diese Erkrankungen bzw. Schädigungen betreffen hierbei bevorzugt das Gehör und/oder die Vestibularfunktion. Begleitende Symptome umfassen unter anderem Hörschäden, Hörverlust, Schwindel, Gleichgewichtsstörungen, Tinnitus, Orientierungslosigkeit, Übelkeit und Erbrechen.

Somit betrifft ein Aspekt der vorliegenden Erfindung die Verbindungen der allgemeinen Formel (I) (bzw. die Fluor-9-methyl-β-carboline der allgemeinen Formel (I)) zur Verwendung als Medikament. Ebenso betrifft die vorliegende Erfindung die Verbindung 6-Fluor-9-methyl-β-carbolin zur Verwendung als Medikament. Ebenso betrifft die vorliegende Erfindung die Verbindung 7-Fluor-9-methyl-β-carbolin zur Verwendung als Medikament. Die erfindungsgemäßen Verbindungen, insbesondere das 7-Fluor-9-methyl-β-carbolin und besonders bevorzugt das 6-Fluor-9-methyl-β-carbolin, sind besonders geeignet zur Behandlung von Erkrankungen und/oder Schädigungen des Innenohres, insbesondere Innenohrschwerhörigkeit. Bei den Erkrankungen und/oder Schädigungen des Innenohres handelt es sich dabei bevorzugt um akute und chronische Erkrankungen des Innenohres. Zu den akuten Erkrankungen des Innenohres zählen unter anderem Knalltrauma, Explosionstrauma des Ohres, Hörsturz und Trauma während der Implantation von Innenohrprothesen (Insertionstrauma). Die chronischen Erkrankungen des Innenohres umfassen unter anderem chronisches Schalltrauma (Innenohrschwerhörigkeit durch chronische Lärmexposition), Presbyakusis (Altersschwerhörigkeit), Otosklerose und die Meniere'sche Erkrankung. Der Begriff Innenohrschwerhörigkeit (auch als cochleäre bzw. sensorische Schwerhörigkeit bezeichnet) umfasst sowohl die Schwerhörigkeit durch chronische Lärmexposition (auch als chronische Lärmschwerhörigkeit oder chronisches Lärmtrauma bezeichnet) als auch die Presbyakusis (Altersschwerhörigkeit).

Ein weiterer Aspekt der vorliegenden Erfindung ist daher gerichtet auf eine Verbindung der allgemeinen Formel (I), insbesondere das 7-Fluor-9-methyl-β-carbolin und besonders bevorzugt das 6-Fluor-9-methyl-β-carbolin, zur Verwendung in der Behandlung von Erkrankungen und/oder Schädigungen des Innenohres, wobei es sich bei den Erkrankungen und/oder Schädigungen des Innenohres um akute Erkrankungen des Innenohres handelt, ausgewählt aus der Gruppe umfassend Knalltrauma, Explosionstrauma des Ohres, Hörsturz und Insertionstrauma.

Ein weiterer Aspekt der vorliegenden Erfindung ist daher gerichtet auf eine Verbindung der allgemeinen Formel (I), insbesondere das 7-Fluor-9-methyl-β-carbolin und besonders bevorzugt das 6-Fluor-9-methyl-β-carbolin, zur Verwendung in der Behandlung von Erkrankungen und/oder Schädigungen des Innenohres, wobei es sich bei den Erkrankungen und/oder Schädigungen des Innenohres um chronische Erkrankungen des Innenohres handelt, ausgewählt aus der Gruppe umfassend chronisches Schalltrauma, Tinnitus und Presbyakusis.

Zudem sind die erfindungsgemäßen Verbindungen zur Behandlung von Schwindel und Gleichgewichtsstörungen verursacht durch Hörschäden oder Ohrerkrankungen, und insbesondere verursacht durch Schäden des Gleichgewichtssinnes im Ohr geeignet sowie zur Steigerung des Gedächtnisses, zur Steigerung der Lernfähigkeit und zur Steigerung der Merkfähigkeit, insbesondere der Merkfähigkeit beim Kurzzeitgedächtnis als auch beim Langzeitgedächtnis.

Erkrankungen und Schädigungen des Innenohrs können zudem durch ototoxische Substanzen wie Antibiotika und/oder Zytostatika ausgelöst werden. Die erfindungsgemäßen Verbindungen, insbesondere das 7-Fluor-9-methyl-β-carbolin und besonders bevorzugt das 6-Fluor-9-methyl-β-carbolin, sind geeignet zur Behandlung von Erkrankungen und/oder Schädigungen des Innenohres, die dadurch gekennzeichnet sind, dass die Schädigung des Innenohrs durch ototoxische Substanzen bedingt ist. Bei den ototoxischen Substanzen handelt es sich dabei bevorzugt um Antibiotika und/oder Zytostatika, besonders bevorzugt aus der Gruppe umfassend oder bestehend aus: Ampicillin, Bacampicillin, Carbenicillin, Indanyl, Mezlocillin, Piperacillin, Ticarcillin, Amoxicillin-Clavulansäure, Ampicillin-Sulbactam, Benzylpenicillin, Cloxacillin, Dicloxacillin, Methicillin, Oxacillin, Penicillin G, Penicillin V, Piperacillin, Tazobactam, Ticarcillin, Clavulansäure, Nafcillin, Cephalosporin, Cefadroxil, Cefazolin, Cephalexin, Cephalothin, Cephapirin, Cephradin, Cefaclor, Cefamandol, Cefonicid, Cefotetan, Cefoxitin, Cefprozil, Ceftmetazol, Cefuroxim, loracarbef, Cefdinir, Ceftibuten, Cefoperazon, Cefixim, Cefotaxim, Cefpodoxim Proxetil, Ceftazidim, Ceftizoxim, Ceftriaxon, Cefepim, Azithromycin, Clarithromycin, Clindamycin, Dirithromycin, Erythromycin, Roxithromycin, Telithromycin, Cethromycin, Spiramycin, Ansymacin, Oelandomycin, Lincomycin, Troleandomycin, Cinoxacin, Ciprofloxacin, Enoxacin, Gatifloxacin, Grepafloxacin, Levofloxacin, lomefloxacin, Moxifloxacin, Nalidixinsäure, Norfloxacin, Ofloxacin, Sparfloxacin, Trovafloxacin, Oxolininsäure, Gemifloxacin, Perfloxacin, Imipenem-Cilastatin, Meropenem, Aztreonam, Amikacin, Gentamicin, Kanamycin, Neomycin, Netilmicin, Streptomycin, Tobramycin, Paromomycin, Teicoplanin, Vancomycin, Demeclocyclin, Doxycyclin, Methacyclin, Minocyclin, Oxytetracyclin, Tetracyclin, Chlortetracyclin, Tigecyclin, Mafenid, Silbersulfadiazin, Sulfacetamid, Sulfadiazin, Sulfamethoxazol, Sulfasalazin, Sulfisoxazole, Trimethoprim-Sulfamethoxazol, Sulfamethizol, Rifabutin, Rifampin, Rifapentin, Linezolid, Streptogramins, Quinopristin, Dalfopristin, Bacitracin, Chloramphenicol, Fosfomycin, Isoniazid, Methenamin, Metronidazol, Mupirocin, Nitrofurantoin, Nitrofurazon, Novobiocin, Polymyxin, Spectinomycin, Trimethoprim, Colistin, Cycloserin, Capreomycin, Ethionamide, Pyrazinamide, Para-Aminosalicyclsäure, Erythromycin-2-Acetat, Erythromycin-2-Stearat, Erythromycinestolat, Erythromycinethylsuccinat, Erythromycinglutamat, Rifampicin, Miconazol, Ketoconazol, Clotrimazol, Econazol, Bifonazol, Butoconazol, Fenticonazol, Isoconazol, Oxiconazol, Sertaconazol, Sulconazol, Tioconazol, Fluconazol, Itroconauzol, Isavuconazol, Ravuconazol, Posaconazol, Voriconazol, Teraconazol, Abfungin, Terbinafin, Amorolfin, Naftifin, Butenafine, Anidulafungin, Caspofungin, Micafungin, Benzoesäure, Ciclopiroxolamin, Tolnaftat, 5-Fluorocytosin, Griseofulvin, Haloprogin, Fusidinsäure, Gramicidin, Pristinamycin, Ramoplanin, Tyrotricin, Natamycin, Rimocidin, Filipin, Nystatin, Amphotericin B, Candicin, und Hamycin.

Folglich ist ein weiterer Aspekt der vorliegenden Erfindung gerichtet auf eine Verbindung der allgemeinen Formel (I), insbesondere das 7-Fluor-9-methyl-β-carbolin und besonders bevorzugt das 6-Fluor-9-methyl-β-carbolin, zur Verwendung in der Behandlung von Erkrankungen und/oder Schädigungen des Innenohres, wobei die Schädigung des Innenohrs durch ototoxische Substanzen bedingt ist.

Ein weiterer Aspekt der vorliegenden Erfindung betrifft die Verwendung der erfindungsgemäßen Verbindungen, insbesondere das 7-Fluor-9-methyl-β-carbolin und besonders bevorzugt das 6-Fluor-9-methyl-β-carbolin, zur Herstellung eines Medikaments zur Behandlung von Erkrankungen und/oder Schädigungen des Innenohres. Eine Ausführungsform der vorliegenden Erfindung betrifft eine Methode zur Behandlung von Erkrankungen und/oder Schädigungen des Innenohres durch Verabreichung einer Verbindung der allgemeinen Formel (I), bevorzugt von 7-Fluor-9-methyl-β-carbolin (Ib) und besonders bevorzugt von 6-Fluor-9-methyl-β-carbolin (Ia).

Zusätzlich soll an dieser Stelle ein zusätzlicher interessanter Aspekt aufgegriffen werden: Wir haben in unseren Untersuchungen auf zellulärer Ebene gefunden, dass das 6-Fluor-9-Methyl-β-carbolin (6F9MβC) die Konzentration von CREB um den Faktor 8 erhöht und die Konzentration von BDNF (*brain derived neurotrophic factor*) bis zu dreifach erhöht. Diese Erkenntnisse legen nahe, dass das erfindungsgemäße 6-Fluor-9-Methyl-β-carbolin (6F9MβC) nicht nur bei der Behandlung von Erkrankungen und/oder Schädigungen des Innenohres sinnvoll einzusetzen ist, sondern auch das Lernen und Gedächtnis fördert.

Die dem Lernen und Gedächtnis zugrunde liegenden Mechanismen sind äußerst komplex. Sie können auf molekularer und zellulärer Ebene, auf der Ebene der Systembiologie und der der Psychologie betrachtet werden. Einer der Schlüsselprozesse für die Gedächtnisbildung auf molekularer Ebene ist die Aktivierung des cAMP response element binding proteins (CREB). Diese führt zur Neubildung von Synapsen, ein Vorgang, an den die Gedächtnisbildung gekoppelt ist. Dieser Vorgang wird durch viele Faktoren reguliert. Eine positive Verstärkung wird durch den brain BDNF und seinen Rezeptor TRKB induziert. In verhaltensbiologischen Experimenten (siehe Beispiel 9) konnte am Tiermodell Ratte gezeigt werden, dass eine intraperitoneale Verabreichung des erfindungsgemäßen 6-Fluor-9-Methyl-β-carbolins (6F9MβC) im Vergleich zu unbehandelten Kontroll-Tieren Lernprozesse beschleunigt und das Gedächtnis verbessert.

Ein weiterer Aspekt der vorliegenden Erfindung ist daher gerichtet auf eine Verbindung der allgemeinen Formel (I), insbesondere das 7-Fluor-9-methyl-β-carbolin und besonders bevorzugt das 6-Fluor-9-methyl-β-carbolin zur Verwendung in der Verbesserung von Lernen und Gedächtnis oder zur Steigerung des Gedächtnisses, in der Steigerung der Lernfähigkeit und zur Steigerung der Merkfähigkeit.

Ein zusätzlicher Aspekt der vorliegenden Erfindung betrifft die Verwendung der erfindungsgemäßen Verbindungen, insbesondere das 7-Fluor-9-methyl-β-carbolin und besonders bevorzugt das 6-Fluor-9-methyl-β-carbolin, zur Herstellung einer pharmazeutischen Formulierung zur Verbesserung von von Lernen und Gedächtnis. Eine Ausführungsform der vorliegenden Erfindung betrifft eine Methode zur Verbesserung von von Lernen und Gedächtnis durch Verabreichung einer Verbindung der allgemeinen Formel (I), bevorzugt von 7-Fluor-9-methyl-β-carbolin (Ib) und besonders bevorzugt von 6-Fluor-9-methyl-β-carbolin (la).

Ein weiterer Aspekt der vorliegenden Erfindung ist daher gerichtet auf eine Verbindung der allgemeinen Formel (I), insbesondere das 7-Fluor-9-methyl-β-carbolin und besonders bevorzugt das 6-Fluor-9-methyl-β-carbolin zur Verwendung in der Verbesserung von Lernprozessen und/oder Gedächtnisprozessen.

Ein zusätzlicher Aspekt der vorliegenden Erfindung betrifft die Verwendung der erfindungsgemäßen Verbindungen, insbesondere das 7-Fluor-9-methyl-β-carbolin und besonders bevorzugt das 6-Fluor-9-methyl-β-carbolin, zur Herstellung einer pharmazeutischen Formulierung zur Verbesserung von Lernprozessen und/oder Gedächtnisprozessen. Eine Ausführungsform der vorliegenden Erfindung betrifft eine Methode zur Verbesserung von Lernprozessen und/oder Gedächtnisprozessen durch Verabreichung einer Verbindung der allgemeinen Formel (I), bevorzugt von 7-Fluor-9-methyl-β-carbolin (Ib) und besonders bevorzugt von 6-Fluor-9-methyl-β-carbolin (la).

Ein weiterer Aspekt der vorliegenden Erfindung betrifft pharmazeutische Zusammensetzungen in Form von Tropfen, Salben, Sprays, Liposomen, Gelen, Emulsionen oder Injektionslösungen enthaltend mindestens eine der erfindungsgemäßen Verbindungen (bzw. mindestens eine Verbindung der allgemeinen Formel (I)).

Ein weiterer Aspekt der vorliegenden Erfindung betrifft pharmazeutische Zusammensetzungen in Form von Tropfen, Salben, Sprays, Liposomen, Gelen, Emulsionen oder Injektionslösungen enthaltend eine oder mehrere der erfindungsgemäßen Verbindungen (bzw. eine oder mehrere Verbindungen der allgemeinen Formel (I)).

Noch ein weiterer Aspekt der vorliegenden Erfindung betrifft pharmazeutische Zusammensetzungen in Form von Tropfen, Salben, Sprays, Liposomen, Gelen, Emulsionen oder Injektionslösungen enthaltend 6-Fluor-9-methyl-β-carbolin.

Ein zusätzlicher Aspekt der vorliegenden Erfindung betrifft pharmazeutische Zusammensetzungen in Form von Tropfen, Salben, Sprays, Liposomen, Gelen, Emulsionen oder Injektionslösungen enthaltend 7-Fluor-9-methyl-β-carbolin.

Noch ein weiterer Aspekt der vorliegenden Erfindung betrifft pharmazeutische Zusammensetzungen in Form von Tropfen, Salben, Sprays, Liposomen, Gelen, Emulsionen oder Injektionslösungen enthaltend 6-Fluor-9-methyl-β-carbolin und 7-Fluor-9-methyl-β-carbolin.

Folglich betrifft die vorliegende Erfindung auch pharmazeutische Zusammensetzungen in Form von Tropfen, Salben, Sprays, Liposomen, Gelen, Emulsionen oder Injektionslösungen enthaltend 6-Fluor-9-methyl-β-carbolin und/oder 7-Fluor-9-methyl-β-carbolin.

Ferner betrifft die vorliegende Erfindung pharmazeutische Zusammensetzungen, welche unter Verwendung mindestens einer der erfindungsgemäßen Verbindungen (bzw. mindestens eine Verbindung der allgemeinen Formel (I)) oder eines Salzes davon hergestellt wurden.

Ebenso betrifft die vorliegende Erfindung pharmazeutische Zusammensetzungen, welche unter Verwendung einer oder mehrerer der erfindungsgemäßen Verbindungen (bzw. einer oder mehrerer Verbindungen der allgemeinen Formel (I)) oder Salzen davon hergestellt wurden.

Ebenso betrifft die vorliegende Erfindung pharmazeutische Zusammensetzungen, welche unter Verwendung der erfindungsgemäßen Verbindung 7-Fluor-9-methyl-β-carbolin oder eines Salzes davon hergestellt wurden.

Ferner betrifft die vorliegende Erfindung pharmazeutische Zusammensetzungen, welche unter Verwendung der erfindungsgemäßen Verbindung 6-Fluor-9-methyl-β-carbolin oder eines Salzes davon hergestellt wurden.

Die vorliegende Erfindung betrifft auch pharmazeutische Zusammensetzungen, welche unter Verwendung der erfindungsgemäßen Verbindung 6-Fluor-9-methyl-β-carbolin oder eines Salzes davon und unter Verwendung der erfindungsgemäßen Verbindung 7-Fluor-9-methyl-β-carbolin oder eines Salzes davon hergestellt wurden.

Gleichermaßen betrifft die vorliegende Erfindung pharmazeutische Zusammensetzungen, welche unter Verwendung der erfindungsgemäßen Verbindung/en 6-Fluor-9-methyl-β-carbolin und/oder 7-Fluor-9-methyl-β-carbolin oder Salzes davon hergestellt wurden.

Neben mindestens einer Verbindung der vorliegenden Erfindung (bzw. einer Verbindung der allgemeinen Formel (I)), insbesondere neben dem 7-Fluor-9-methyl-β-carbolin und besonders bevorzugt neben dem 6-Fluor-9-methyl-β-carbolin, enthalten die pharmazeutischen Zusammensetzungen bevorzugt einen pharmakologisch verträglichen Träger, Hilfsstoff und/oder Lösungsmittel.

Neben dem 6-Fluor-9-methyl-β-carbolin der vorliegenden Erfindung enthalten die pharmazeutischen Zusammensetzungen bevorzugt einen pharmakologisch verträglichen Träger, Hilfsstoff und/oder Lösungsmittel.

Die pharmazeutischen Zusammensetzungen, die erfindungsgemäße Verbindung enthaltend, können in Form von transdermalen Applikationssystemen (Pflaster, Film), Tropfen, Pillen, Tabletten, Filmtabletten, Schichttabletten, Dragees, Gelen, Hydrogelen, Salben, Sirupen, Granulaten, Suppositorien (Zäpfchen), Emulsionen, Dispersionen, Mikrokapseln, Kapseln, Mikroformulierungen, Nanoformulierungen, Liposomen, Pulvern, Pudern, Sprays, Aerosolen, Lösungen, Säften, Suspensionen, Infusionslösungen oder Injektionslösungen hergestellt und verabreicht werden. Bevorzugt sind pharmazeutische Zusammensetzungen in Form von Liposomen, Gelen und Emulsionen. Insbesondere bevorzugt sind Hydrogelformulierungen.

Derartige Zusammensetzungen sind unter anderem für die Inhalation oder die intravenöse, intraperitoneale, intramuskuläre, subkutane, mucokutane, orale, rektale, transdermale, topikale, buccale, intradermale, intragastrale, intrakutane, intranasale, intrabuccale, perkutane, intratympanale oder sublinguale Verabreichung geeignet. Besonders bevorzugt ist die Verabreichung bzw. Injektion in das Mittelohr sowie die topische Verabreichung auf das Trommelfell.

Als pharmakologisch verträgliche Träger können beispielsweise Lactose, Stärke, Sorbitol, Sucrose, Cellulose, Magnesiumstearat, Dicalciumphosphate, Calciumsulfate, Talk, Mannitol, Ethylalkohol und dergleichen eingesetzt werden. Puder als auch Tabletten können zu 5 bis 95 Gew. % aus einem derartigen Träger bestehen.

Ferner können den pharmazeutischen Zusammensetzungen noch Sprengmittel, Farbstoffe, Geschmacksstoffe und/oder Bindemittel zugesetzt werden.

Flüssige Formulierungen umfassen Lösungen, Suspensionen, Sprays und Emulsionen. Beispielsweise Injektionslösungen auf Wasserbasis oder Wasser-Propylenglycol-Basis für parenterale Injektionen. Für die Zubereitung von Suppositorien werden bevorzugt niedrigschmelzende Wachse, Fettsäureester und Glyceride eingesetzt.

Kapseln werden beispielsweise aus Methylcellulose, Polyvinylalkoholen oder denaturierter Gelatine oder Stärke hergestellt. Als Sprengmittel können Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, Natrium-Carboxymethylcellulose, mikrokristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Mais, Reis oder Kartoffeln, sowohl natürliche als auch synthetische Gummis wie Akaziengummi oder Guar-Gummi, Gelatine, Tragacanth, Alginsäure, Natriumalginat, Ammonium-Calcium-Alginat, Methylcellulose, Natrium-Carboxymethylcellulose, Hydroxypropyl-methylcellulose, Polyvinylpyrrolidon, Polyethylenglycol, Wachse sowie anorganische Verbindungen wie Magnesium-Aluminum-Silicate zugesetzt werden. Die Bindemittel können in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol, Borsäure und Aminosäuren wie Leucin eingesetzt werden. Derartige Gleitmittel können in Mengen von 0,05 bis 15 Gew.-% verwendet werden.

Somit sind alle pharmazeutischen Formulierungen bevorzugt, welche geeignet sind, den Wirkstoff lokal an der Rundfenstermembran zu applizieren. Ferner bevorzugt ist, wenn die pharmazeutischen Formulierungen einen Membranpenetrationsförderer enthalten, der den Durchtritt der erfindungsgemäßen Verbindung der allgemeinen Formel (I), inbesondere des 7-Fluor-9-methyl-β-carbolins und besonders bevorzugt des 6-Fluor-9-methyl-β-carbolins durch die Rundfenstermembran unterstützt. Dementsprechend sind flüssige oder gelförmige Formulierungen insbesondere bevorzugt. Natürlich ist es auch möglich, den Wirkstoff oral zu applizieren.

Pharmazeutische Zusammensetzungen für eine beliebige Art der Verabreichung von Verbindungen der allgemeinen Formel (I) enthalten eine für den therapeutischen Effekt ausreichende Menge an der/den Verbindung/en der allgemeinen Formel (I) und, falls nötig, anorganische oder organische, feste oder flüssige pharmazeutisch verträgliche Trägerstoffe. Pharmazeutische Zusammensetzungen, die für eine topische Verabreichung im Mittelohr geeignet sind, beinhalten wässrige Lösungen oder Suspensionen, die vor der Verabreichung im Mittelohr hergestellt werden können, z. B. im Fall von lyophilisierten Formulierungen, die die Verbindung/en der allgemeinen Formel (I), eventuell zusammen mit einem Träger enthalten.

Pharmazeutische Zusammensetzungen für eine beliebige Art der Verabreichung von 7-Fluor-9-methyl-β-carbolin enthalten eine für den therapeutischen Effekt ausreichende Menge an 7-Fluor-9-methyl-β-carbolin und, falls nötig, anorganische oder organische, feste oder flüssige pharmazeutisch verträgliche Trägerstoffe. Pharmazeutische Zusammensetzungen, die für eine topische Verabreichung im Mittelohr geeignet sind, beinhalten wässrige Lösungen oder Suspensionen, die vor der Verabreichung im Mittelohr hergestellt werden können, z. B. im Fall von lyophilisierten Formulierungen, die 7-Fluor-9-methyl-β-carbolin alleine oder zusammen mit einem Träger enthalten.

Pharmazeutische Zusammensetzungen für eine beliebige Art der Verabreichung von 6-Fluor-9-methyl-β-carbolin enthalten eine für den therapeutischen Effekt ausreichende Menge an 6-Fluor-9-methyl-β-carbolin und, falls nötig, anorganische oder organische, feste oder flüssige pharmazeutisch verträgliche Trägerstoffe. Pharmazeutische Zusammensetzungen, die für eine topische Verabreichung im Mittelohr geeignet sind, beinhalten wässrige Lösungen oder Suspensionen, die vor der Verabreichung im Mittelohr hergestellt werden können, z. B. im Fall von lyophilisierten Formulierungen, die 6-Fluor-9-methyl-β-carbolin alleine oder zusammen mit einem Träger enthalten.

Die pharmazeutischen Zusammensetzungen schließen weiterhin Gele, die biologische abbaubar oder nicht-biologisch abbaubar, wässrig oder nicht-wässrig oder Mikrosphären basiert sind ein. Beispiele für solche Gele beinhalten Polyoxamere, Hyaluronate, Xyloglucane, Chitosane, Polyester, Polylactide, Polyglycolide oder deren Co-Polymer PLGA, Saccharoseacetatisobutyrat und Gylcerinmonooleat. Pharmazeutische Zusammensetzungen, die für eine enterale oder parenterale Verabreichung geeignet sind, beinhalten Tabletten oder Gelatinekapseln oder wässrige Lösungen oder Suspension wie oben beschrieben.

Die pharmazeutischen Zusammensetzungen können sterilisiert werden und/oder Adjuvantien enthalten, z. B. Konservierungsstoffe, Stabilisatoren, Feuchthaltemittel und/oder Emulgatoren, Salze für die Regulierung des osmotischen Drucks und/oder Puffer. Die erfindungsgemäße pharmazeutische Zusammensetzung kann, falls gewünscht, weitere Wirkstoffe enthalten. Diese pharmazeutischen Zusammensetzungen können durch eine beliebige Methode, die aus dem Stand der Technik bekannt ist, hergestellt werden, z. B. durch herkömmliche Methoden wie Vermischung, Granulierung, Konfektionierung, Lösung und Lyophilisierung und können von ungefähr 0,01 bis 100 Gew. - Prozent, bevorzugt zwischen 0,1 und 50 Gew. - Prozent, bei Lyophilisaten bis zu 100 Gew. - Prozent an 6-Fluor-9-methyl-β-carbolin enthalten. Diese pharmazeutischen Zusammensetzungen können auch von ungefähr 0,01 bis 100 Gew. - Prozent, bevorzugt zwischen 0,1 und 50 Gew. - Prozent, bei Lyophilisaten bis zu 100 Gew. - Prozent an 7-Fluor-9-methyl-β-carbolin enthalten. Diese pharmazeutischen Zusammensetzungen können auch von ungefähr 0,01 bis 100 Gew. - Prozent, bevorzugt zwischen 0,1 und 50 Gew. - Prozent, bei Lyophilisaten bis zu 100 Gew. - Prozent an Verbindungen der allgemeinen Formel (I) enthalten.

Bei einer bevorzugten Ausführungsform wird die erfindungsgemäße pharmazeutische Zusammensetzung für eine topische Verabreichung formuliert. Geeignete Träger für eine otogene Verabreichung sind organische oder anorganische Substanzen, die pharmazeutisch verträglich sind und die nicht mit einer erfindungsgemäßen Verbindung und/oder den weiteren Wirkstoffen reagieren, z. B. Kochsalz, Alkohole, pflanzliche Öle, Benzylalkohole, Alkylglycole, Polyethylenglycole, Glycerintriacetate, Gelatine, Kohlenhydrate wie Lactose oder Stärke, Magnesiumcarbonat (Magnesia, *Chalk*), Stearat (Wachse), Talk und Petrolatum (Vaseline). Die beschriebenen Zusammensetzungen können sterilisiert werden und/oder Hilfsstoffe wie Gleitmittel, Konservierungsstoffe wie Thiomersal (z. B. 50 Gew.-%), Stabilisatoren und/oder Feuchthaltemittel, Emulgatoren, Salze zur Beeinflussung des osmotischen Drucks, Puffersubstanzen, Farbstoffe und/oder Geschmacksstoffe enthalten. Diese Zusammensetzungen können gegebenenfalls auch einen oder mehrere weitere Wirkstoffe enthalten. Erfindungsgemäße otogene Zusammensetzungen können verschiedene Stoffe und/oder Substanzen umfassen, z.B. andere biologisch aktive Substanzen wie Antibiotika, antiinflammatorische Wirkstoffe wie Steroide, Cortisone, Analgetika, Antipyrine, Benzocaine, Procaine.

Erfindungsgemäße Zusammensetzungen für die topische Verabreichung können andere pharmazeutische verträgliche Stoffe und/oder Substanzen enthalten. In bevorzugten Ausführungsformen der vorliegenden Erfindung wird ein topischer Exzipient ausgewählt, der die Abgabe von Verbindungen der allgemeinen Formel (I), insbesondere 7-Fluor-9-methyl-β-carbolin und besonders bevorzugt 6-Fluor-9-methyl-β-carbolin, und des gegebenenfalls weiteren Wirkstoffs oder der Wirkstoffe an das Blutkreislaufsystem oder an das Zentralnervensystem nicht verstärkt, wenn dieser am Ohr, im Mittelohr oder im Gehörgang verabreicht wird. Mögliche Trägerstoffe beinhalten Hydrocarbonsäuren, wasserfreie Absorptionsmittel wie hydrophiles Petrolatum (Vaseline) und wasserfreies Lanolin (z. B. Aquaphor^{®}) und Mittel auf Basis von Wasser-Öl-Emulsionen wie Lanolin und *Cold Cream.* Mehr bevorzugt sind Trägerstoffe, die im wesentlichen nicht-ausschließend sind und beinhalten üblicherweise diejenigen Trägerstoffe, die wasserlöslich sind, wie auch Stoffe auf Basis von Öl-in-Wasser-Emulsionen (Cremes oder hydrophile Salben) und Stoffe auf wasserlöslicher Basis wie auf Polyethylenglycol basierende Trägerstoffe und wässrige Lösungen, die mit verschiedenen Stoffen wie Methylcellulose, Hydroxyethylcellulose und Hydroxpropylmethylcellulose geliert wurden.

Bei oraler Verabreichung in Form von Tabletten oder Kapseln kann eine erfindungsgemäße Verbindung gemischt werden mit nicht-toxischen pharmazeutisch verträglichen Hilfsstoffen ausgewählt aus der Liste umfassend Bindemittel wie vorgelierte Maisstärke, Polyvinylpyrrolidon oder Hydroxypropylmethylcellulose; Füllstoffe wie Lactose, Saccharose, Glucose, Mannitol, Sorbitol und andere reduzierende und nicht-reduzierende Zucker, mikrokristalline Cellulose, Calciumsulfat oder Calciumhydrogenphosphat; Gleitmittel wie Magnesiumstearat, Talkum oder Silica, Stearinsäure, Natriumstearylfumarat, Glycerylbehenat, Calciumstearat und ähnliche; Zerfallsbeschleuniger wie Kartoffelstärke oder Natriumglycolat-Stärke; Befeuchtungsmittel wie Natriumdodecylsulfat; Farbstoffe; Geschmacksstoffe; Gelatine; Süßstoffe; natürliche und syntheische Gummis wie Gummi arabicum, Tragacanth oder Alginate; Puffersalze; Carboxymethylcellulose; Polyethylenglycol; Wachse und ähnliche.

Die Tabletten können mit einer konzentrierten Zuckerlösung umhüllt sein, die zum Beispiel Gummi arabicum, Gelatine, Talcum, Titandioxid und ähnliches enthalten kann. Bei einer anderen Ausführungsform können die Tabletten mit einem Polymer umhüllt sein, das sich in einem leichtflüchtigen organischen Lösungsmittel oder einer Mixtur an organischen Lösungsmitteln löst. In bevorzugten Ausführungsformen sind die erfindungsgemäßen Verbindungen der allgemeinen Formel (I), insbesondere das 7-Fluor-9-methyl-β-carbolin und besonders bevorzugt das 6-Fluor-9-methyl-β-carbolin als Tablette zur sofortigen Freigabe oder als Tablette in Retardform formuliert. Dosierungsformen zur sofortigen Freigabe erlauben die Freisetzung eines Großteils oder der Gesamtmenge an einer Verbindung der allgemeinen Formel (I) innerhalb einer kurzen Zeitspanne wie 60 Minuten oder weniger und ermöglichen die rasche Absorption der erfindungsgemäßen Verbindung. Retardformen erlauben die verzögerte Freisetzung über einen längeren Zeitraum, um einen therapeutisch wirksamen Plasmaspiegel an einer Verbindung der allgemeinen Formel (I) zu erreichen und/oder diesen therapeutisch wirksamen Plasmaspiegel über einen längeren Zeitraum stabil zu halten und/oder andere pharmakokinetische Eigenschaften der Verbindungen der allgemeinen Formel (I), insbesondere des 7-Fluor-9-methyl-β-carbolins und besonders bevorzugt des 6-Fluor-9-methyl-β-carbolins, zu modifizieren.

Zur Formulierung von Weichgelatinekapseln kann eine erfindungsgemäße Verbindung zum Beispiel mit einem pflanzlichen Öl oder Polyethylenglycol vermengt werden. Hartgelatinekapseln können eine erfindungsgemäße Verbindung in Granulatform enthalten unter Verwendung von entweder den oben genannten Hilfsstoffen für Tabletten wie Lactose, Saccharose, Mannitol, Stärken wie Kartoffelstärke, Maisstärke oder Amylopectin, Cellulosederivate oder Gelatine. Auch flüssige oder semiflüssige Formen einer erfindungsgemäßen Verbindung können in die Hartgelatinekapseln gefüllt werden.

Die erfindungsgemäßen Verbindungen der allgemeinen Formel (I), insbesondere das 7-Fluor-9-methyl-β-carbolin und besonders bevorzugt das 6-Fluor-9-methyl-β-carbolin, können auch in Mikrokügelchen oder Mikrokapseln, die z. B. aus Polyglycolsäure/Milchsäure (PGLA) hergestellt sind, eingebracht werden. Bioverträgliche Polymere, ausgewählt aus der Liste umfassend Polymilchsäure, Polyglycolsäure, Copolymere von Polymilchsäure und Polyglycolsäure, Poly-ε-Caprolacton, Polyhydroxybuttersäure, Polyoethoester, Polyacetale, Polyhydropurane, Polycyanoacrylate und vernetzte oder amphipathische Blockcopolymere von Hydrogelen, können verwendet werden, um eine kontrollierte Freisetzung einer erfindungsgemäß Verbindung aus den pharmazeutischen Zusammensetzungen der vorliegenden Erfindung zu erreichen.

In einer weiteren Ausführungsform der Erfindung werden die Verbindungen der allgemeinen Formel (I), insbesondere das 7-Fluor-9-methyl-β-carbolin und besonders bevorzugt das 6-Fluor-9-methyl-β-carbolin, als eine flüssige Formulierung zur oralen Verabreichung bereitgestellt. Flüssige Zubereitungen für die orale Verabreichung können in Form von Lösungen, Sirupen, Emulsionen oder Suspensionen bereitgestellt werden. Alternativ können die flüssigen Formulierungen zur oralen Verabreichung durch Rekonstitution der trockenen Formulierung zur oralen Verabreichung mit Wasser oder einem anderen geeignetem Trägerstoff vor der Anwendung hergestellt werden. Zubereitungen zur oralen Verabreichung können so formuliert werden, dass eine kontrollierte oder retardierte Freisetzung einer erfindungsgemäßen Verbindung und gegebenenfalls weiterer Wirkstoffe erreicht werden kann.

Bei oraler Verabreichung in flüssiger Form kann eine erfindungsgemäße Verbindung mit nicht-toxischen pharmazeutisch verträglichen inerten Trägerstoffen wie Ethanol, Glycerin, Wasser; Suspensionsmitteln wie Sorbitolsyrup, Cellulosederivate oder gehärteten essbaren Fetten; Emulgatoren wie Lecithin oder Gummi arabicum; nichtwässrigen Trägerstoffen wie Mandelöl, öligen Estern, Ethanol oder fraktionierten pflanzlichen Ölen; Konservierungsstoffen wie Methyl- oder Propyl-p-hydroxybenzoaten oder Sorbinsäure; und ähnlichen. Stabilisatoren wie z. B. Antioxidantien wie Butylhydroxyanisol, Butylhydroxytoluol, Propylgallat, Natriumascorbat, Zitronensäure können auch zur Stabilisierung der Dosierungsform verwendet werden. Zum Beispiel können die Lösungen ungefähr 0,2 Gew.-% bis ungefähr 20 Gew.-% einer erfindungsgemäßen Verbindung enthalten, wobei die Ausgleichsmasse Zucker und eine Mischung aus Ethanol, Wasser, Glycerin und Propylenglycol sein kann. Optional können diese flüssigen Formulierungen Farbstoffe, Geschmacksstoffe, Saccharin, Carboxylcellulose als Verdickungsmittel und/oder andere Hilfsstoffe enthalten.

Bei einer weiteren Ausführungsform wird eine therapeutisch wirksame Dosis einer erfindungsgemäßen Verbindung über eine Lösung oral verabreicht, wobei die Lösung einen Konservierungsstoff, Süßstoff, Lösungsvermittler und ein Lösungsmittel enthalten kann. Die Lösung zur oralen Verabreichung kann einen oder mehrere Puffer, Geschmackstoffe oder weitere Exzipienten enthalten. Bei einer weiteren Ausführungsform wird Pfefferminze oder ein anderer Geschmacksstoff zu der Lösung der erfindungsgemäßen Verbindung für die orale Verabreichung hinzugefügt.

Für die Verabreichung über Inhalation kann eine erfindungsgemäße Verbindung der in geeigneter Weise verabreicht werden, wie in der Darreichungsform als Aerosolspray mit unter Druck stehenden Packungen oder einem Zerstäuber, unter Verwendung eines geeigneten Treibgases wie Dichlorfluormethan, Trichlorfluormethan, Dichlortetrafluorethan, Kohlendioxid oder eines anderen geeigneten Gases. Bei Verwendung eines unter Druck stehenden Aerosols kann die Dosis dadurch bestimmt werden, dass ein Ventil bereitgestellt wird, um eine abgemessene Menge zu verabreichen. Kapseln und Kartuschen aus z. B. Gelatine für die Verwendung in Inhalatoren oder Insufflatoren können so formuliert werden, dass die Kapseln und Kartuschen eine Pulvermischung der erfindungsgemäßen Verbindung und gegebenenfalls eines weiteren oder mehrerer weiterer Wirkstoffe sowie eine geeignete Pulverbasissubstanz wie Lactose oder Stärke enthalten.

Lösungen für die parenterale Verabreichung durch Injektion können aus einer wässrigen Lösung eines wasserlöslichen pharmazeutisch verträglichen Salzes einer erfindungsgemäßen Verbindung, insbesondere des 7-Fluor-9-methyl-β-carbolins und besonders bevorzugt des 6-Fluor-9-methyl-β-carbolins, mit einer Konzentration von ungefähr 0,5 Gew.-% bis ungefähr 10 Gew.-% hergestellt werden. Diese Lösungen können auch Stabilisatoren und/oder Puffersubstanzen enthalten und können in geeigneter Weise über Ampullen mit verschiedenen Dosiseinheiten bereitgestellt werden.

Somit sind die hier vorgestellten erfindungsgemäßen Verbindungen, insbesondere das 7-Fluor-9-methyl-β-carbolin und besonders bevorzugt das 6-Fluor-9-methyl-β-carbolin, nützlich für die Herstellung von pharmazeutischen Zusammensetzungen zur Behandlung von Erkrankungen und/oder Schädigungen des Innenohrs. Hierbei handelt es sich bei den Erkrankungen und/oder Schädigungen des Innenohrs bevorzugt um akute und/oder chronische Erkrankungen des Innenohrs. Zu den akuten Erkrankungen des Innenohrs gehören unter anderem Knalltrauma, Explosionstrauma des Ohres, Hörsturz und Insertionstrauma. Zu den chronischen Erkrankungen des Innenohrs gehören unter anderem chronisches Schalltrauma, Tinnitus und Presbyakusis. Die Erkrankungen und/oder Schädigungen des Innenohrs können zudem dadurch gekennzeichnet sein, dass diese durch ototoxische Substanzen bedingt sein können.

### Beschreibung der Figuren

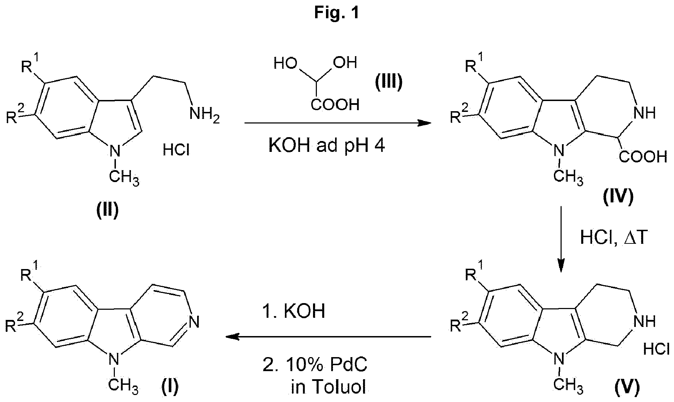

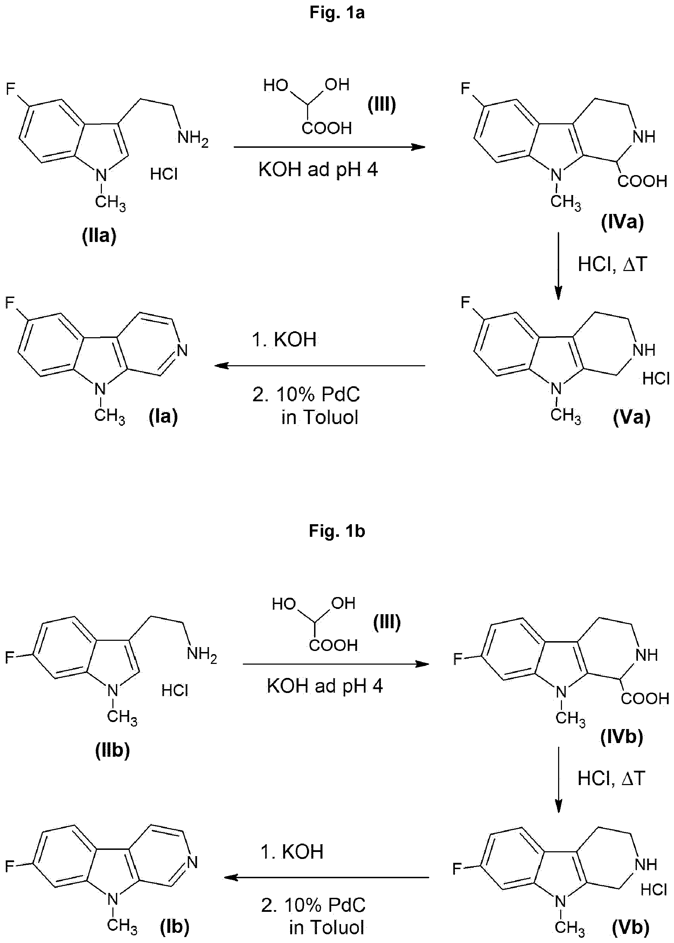

- **Fig.1:**: Reaktionsschema zur dreistufigen Reaktion eines Fluor-1-methyltryptamins (als Hydrochlorid) der allgemeinen Formel (II) und Glyoxalsäurehydrat (III) über das Zwischenprodukt der allgemeinen Formel (IV) und einem Fluor-9-methyl-2,3,4,9-tetrahydro-β-carbolin der allgemeinen Formel (V) zu einem Fluor-9-methyl-β-carbolin der allgemeinen Formel (I). Hierbei gilt entweder, dass R¹ = -F und R² = -H oder -F ist und dies gleichermaßen für die Verbindungen der allgemeinen Formel (I), (II), (IV) und (V) gilt, oder dass R¹ = -H und R² = -F ist und dies gleichermaßen für die Verbindungen der allgemeinen Formel (I), (II), (IV) und (V) gilt.
- **Fig. 1a:**: Reaktionsschema zur dreistufigen Reaktion von 5-Fluor-1-methyltryptamin Hydrochlorid (IIa) und Glyoxalsäurehydrat (III) über das Zwischenprodukt (IVa) und 6-Fluor-9-methyl-2,3,4,9-tetrahydro-β-carbolin (Va) zu 6-Fluor-9-methyl-β-carbolin (la).
- **Fig. 1b:**: Reaktionsschema zur dreistufigen Reaktion von 6-Fluor-1-methyltryptamin Hydrochlorid (IIb) und Glyoxalsäurehydrat (III) über das Zwischenprodukt (IVb) und 7-Fluor-9-methyl-2,3,4,9-tetrahydro-β-carbolin (Vb) zu 7-Fluor-9-methyl-β-carbolin (Ib).
- **Fig. 2:**: SH-SY5Y-Zelllinie 15 Tage Inkubation mit AC102 (6-Fluor-9-methyl-β-carbolin)
- **Fig. 3:**: SH-SY5Y-Zelllinie 15 Tage Inkubation mit AC002 (9-Methyl-β-carbolin)

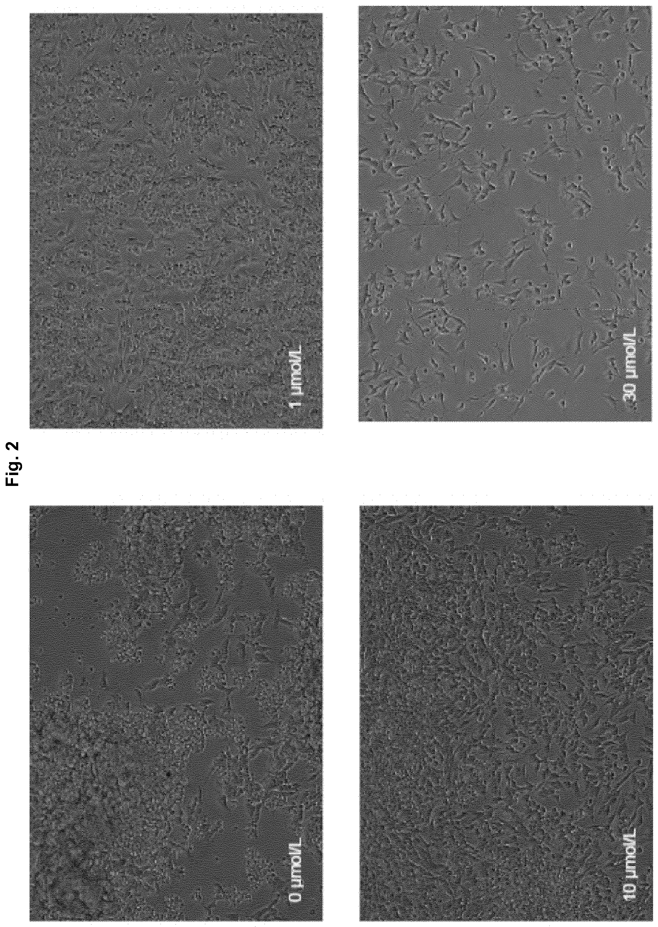

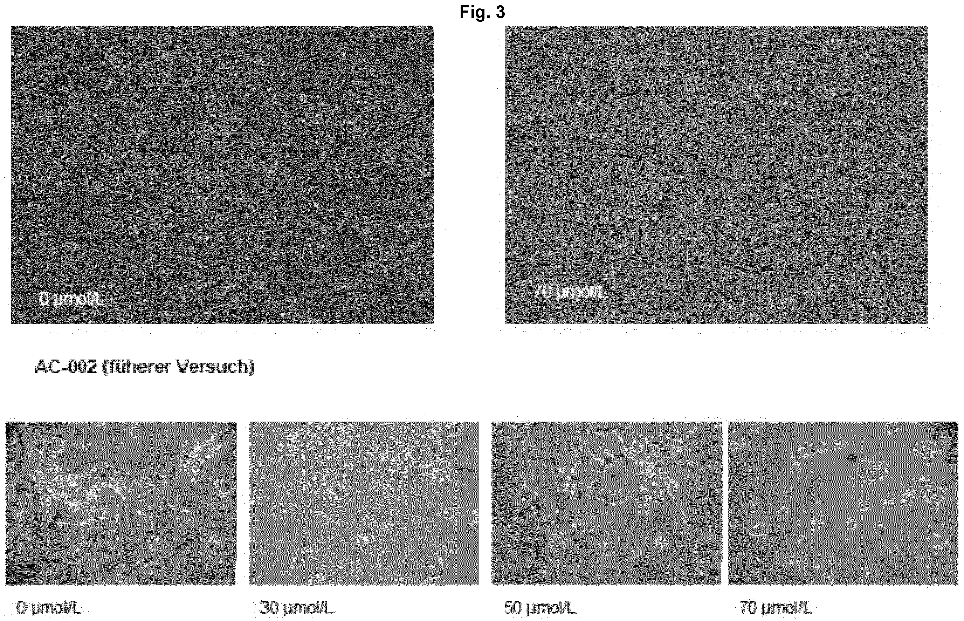

- **Fig. 2 und 3:**: zeigen die Kultur von humanen Neuroblastomzellen (SH-SY5Y), die mit ansteigenden Konzentrationen von 6-Fluor-9-methyl-β-carbolin (AC102) 15 Tage lang exponiert wurden (Fig. 2). Zum Vergleich ist in der Fig. 3 unter denselben Bedingungen die Wirkung von 9-Methyl-β-Carbolin (AC002) dargestellt. Die obere Reihe stammt aus dem aktuellen Experiment mit der Konzentration 70 µmol/L, während die untere Reihe die Konzentrationsabhängigkeit aus einem früheren Experiment zeigt. Die erfindungsgemäße Verbindung (AC102) hemmt die Proliferation der Zellen und induziert die Aussprossung von schlanken Fortsätzen sowie eine Veränderung von Größe und Form der Zellen. Dies ist mit der Induktion einer Differenzierung zu erklären. Besonders deutlich wird diese Wirkung der Verbindung beim Vergleich der Konzentrationen 0 µM (Kontrolle) und 30 µM zu beiden Zeitpunkten.

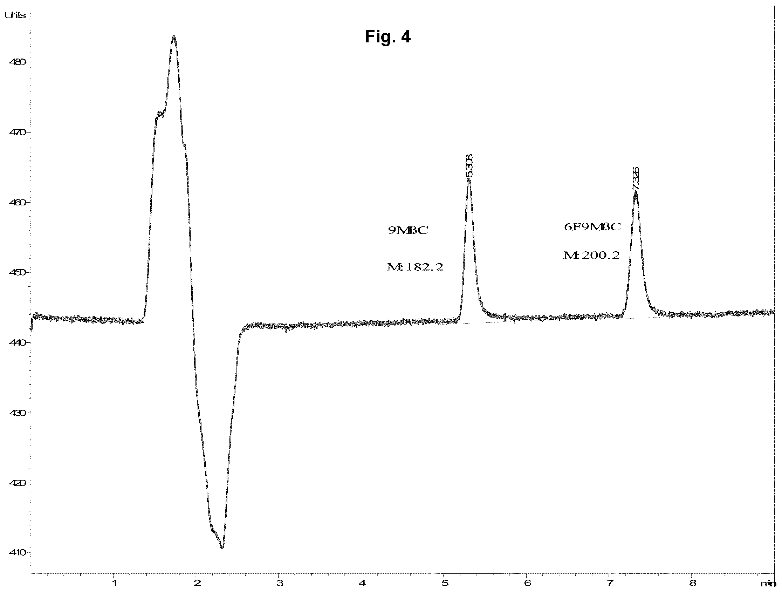

- **Fig.4:**: HPLC Chromatogramm von 9-Methyl-β-carbolin (9MβC, AC002) und 6-Fluor-9-methyl-β-carbolin (6F9MβC, AC102). Die Retentionszeiten betrugen 5,3 bzw. 7,3 Min. Säule: Phenomenex Luna 3uC18 (2) 100A; Eluent: ACN90%/NH₄Ac 40mM [45/55]

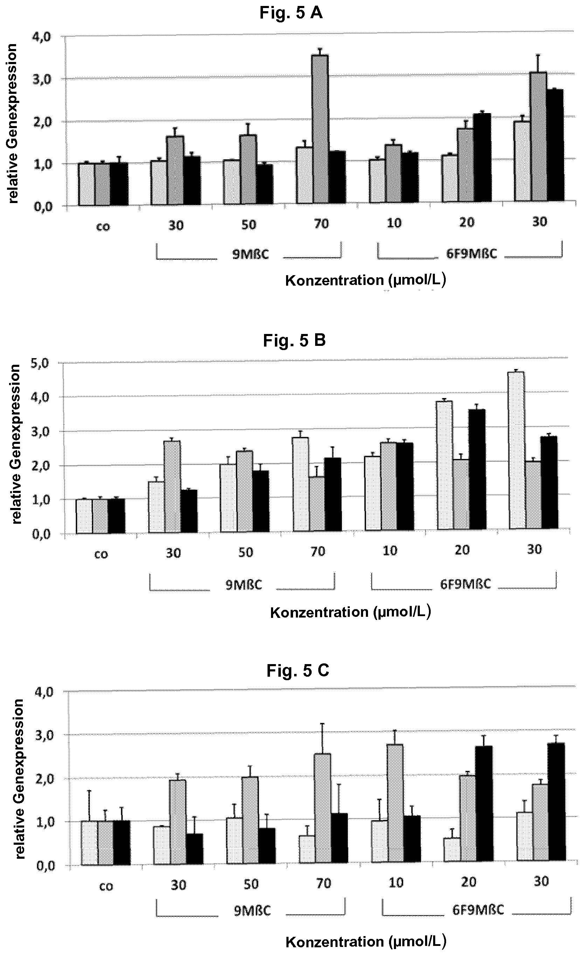

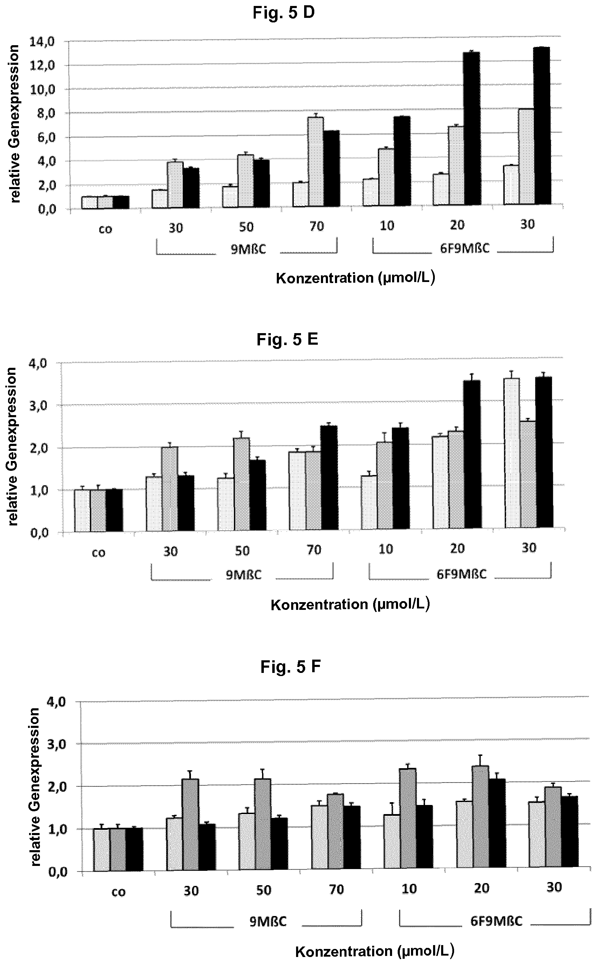

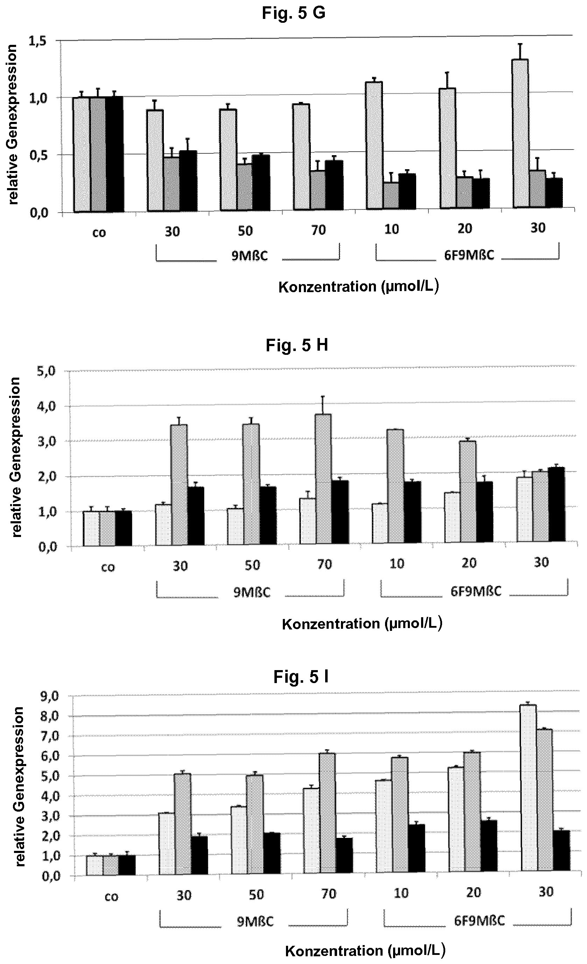

- **Fig.5**: zeigt die relative Genexpression von BDNF (*brain*-*derived neurotrophic factor,* **Fig. 5A**), von Lif (*Leukemia inhibitory factor,* **Fig. 5B**), von NT3 *(Neurotrophin 3,* **Fig. 5C**), von Id2 (*Inhibitor of Differentiation 2,* **Fig. 5D**), von BTG2 (*B*-*cell translocation gene 2,* **Fig. 5E**), von Cbln (*Cerebellin 1 precursor Protein,* **Fig. 5F**), von DRD2S *(kurze Variante des Dopaminrezeptors Subtyp 2,* **Fig. 5H**), von DKK1 (*Dickkopf-related Protein 1,* **Fig. 5I**) sowie den Quotienten Bax/Bcl2 (**Fig. 5G**) jeweils nach 2 Tagen (hellgrauer Balken), 7 Tagen (dunkelgrauer Balken) und 14 Tagen (schwarzer Balken) Exposition von SH-SY5Y-Zellen gegenüber 6F9MβC (10 µM, 20 µM & 30 µM) oder 9MβC (30 µM, 50 µM & 70 µM). Relative Genexpression bedeutet die Genexpression des jeweiligen Faktors relativ zu den Kontrollbedingungen.

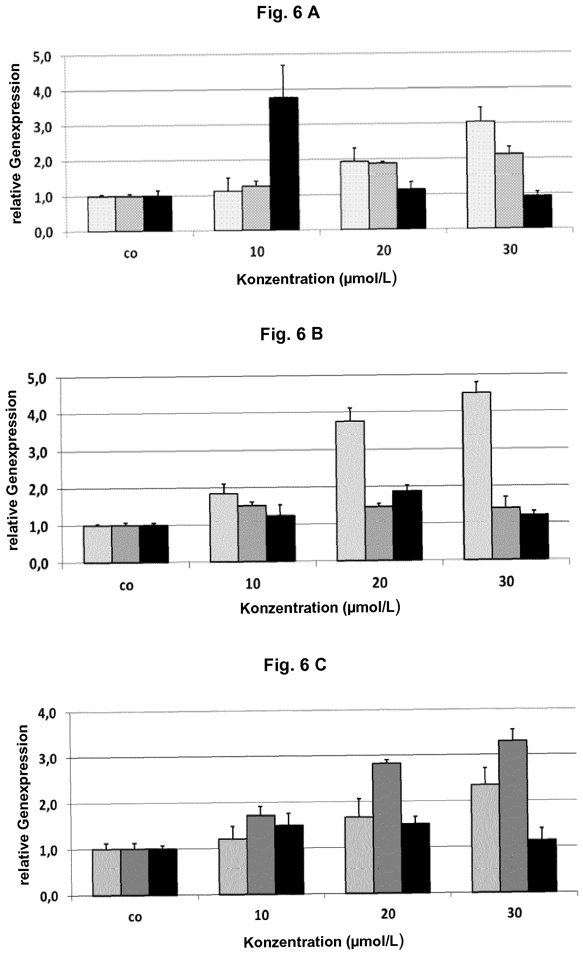

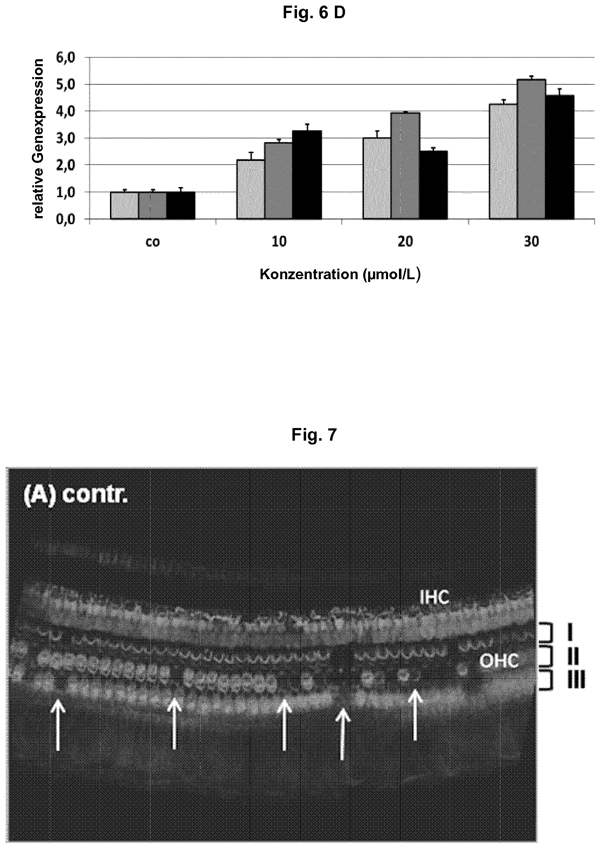

- **Fig.6**: zeigt die relative Genexpression von BDNF (*brain*-*derived neurotrophic factor,* **Fig. 6A**), von Lif (*Leukemia inhibitory factor,* **Fig. 6B**), von DRD2S *(kurze Variante des Dopaminrezeptors Subtyp 2,* **Fig. 6C**) und von DKK1 (*Dickkopf-related Protein 1,* **Fig. 6D**) jeweils nach 2 Tagen (hellgrauer Balken), 7 Tagen (dunkelgrauer Balken) und 14 Tagen (schwarzer Balken) Exposition von SH-SY5Y-Zellen gegenüber 7F9MβC. Relative Genexpression bedeutet die Genexpression des jeweiligen Faktors relativ zu den Kontrollbedingungen.

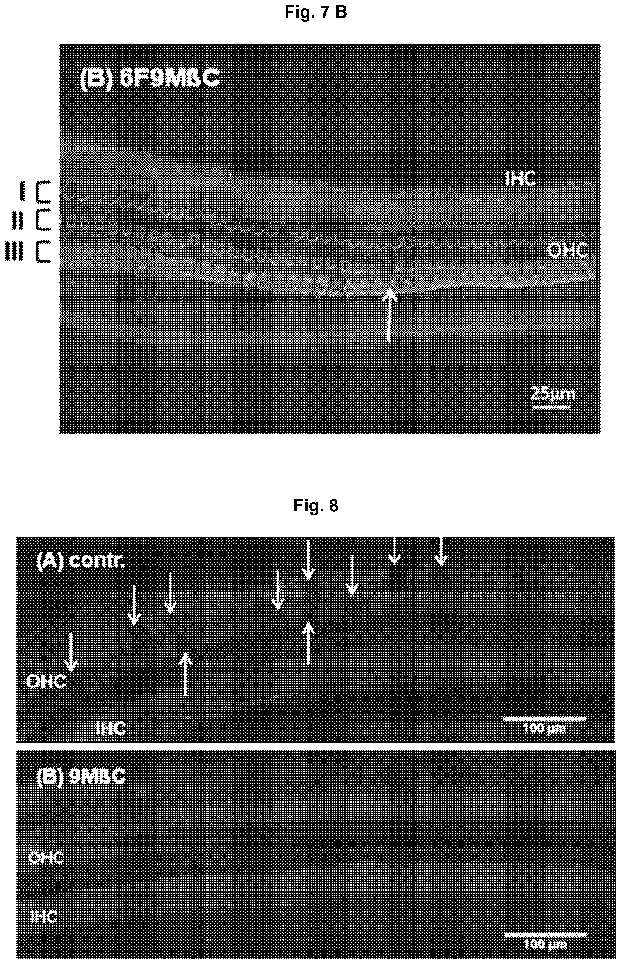

- **Fig.7:**: Fluoreszenzmikroskopische Aufnahmen von Cochleaschnitten nach immunhistochemischer Färbung mit Phalloidin (mit Alexa Fluor^{®} 488 markiert) eines Meerschweinchens nach einem Schalltrauma, wobei ein Ohr mit 6-Fluor-9-methyl-β-carbolin (6F9MβC, insgesamt 0,105 mg pro Ohr) behandelt wurde (**Fig. 7B**) und das zweite unbehandelte Ohr als intra-individuelle Kontrolle (auch als "contr." bezeichnet, **Fig. 7A**) diente. Zu erkennen sind die Inneren Haarzellen (IHC) und die drei Reihen (I, II, II) Äußere Haarzellen (OHC). Abgestorbene Zellen sind durch weiße Pfeile markiert und demonstrieren den schädlichen Effekt des Schalltraumas. Maßstab = 25 µm.
- **Fig.8:**: Fluoreszenzmikroskopische Aufnahmen von Cochleaschnitten nach immunhistochemischer Färbung mit Phalloidin (mit Alexa Fluor^{®} 488 markiert) eines Meerschweinchens nach einem Schalltrauma, wobei ein Ohr mit 9-Methyl-β-carbolin (9MβC, insgesamt 0,2 mg pro Ohr) behandelt wurde (**Fig. 8B**) und das zweite unbehandelte Ohr als intra-individuelle Kontrolle (auch als "contr." bezeichnet, **Fig. 8A**) diente. Zu erkennen sind die Inneren Haarzellen (IHC) und die Äußeren Haarzellen (OHC). Abgestorbene Zellen sind durch weiße Pfeile markiert und demonstrieren den schädlichen Effekt des Schalltraumas. Maßstab = 100 µm.

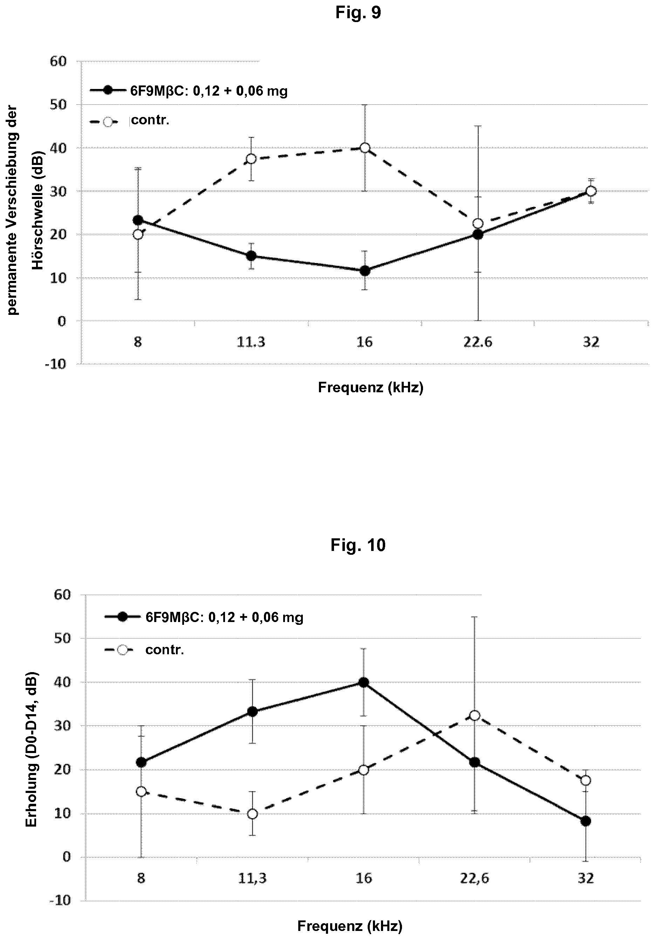

- **Fig. 9**: zeigt die permanente Verschiebung der Hörschwelle am Meerschweinchen nach einem akustisch induzierten Schalltrauma, wobei ein Ohr nach dem Trauma mit 6-Fluor-9-methyl-β-carbolin (6F9MβC, insgesamt 0,18 mg pro Ohr) behandelt wurde und das zweite unbehandelte Ohr als intra-individuelle Kontrolle (contr.) diente. Die Hörschwelle wurde durch Messung akustisch evozierter Hirnstammpotenziale bestimmt, welche 3 Tage vor dem akustischen Schalltrauma (D-3), 30 Minuten nach dem akustischen Schalltrauma (D0) sowie 14 Tage nach dem akustischen Schalltrauma (D14) gemessen wurden. Die permanente Verschiebung der Hörschwelle berechnet sich aus den Messwerten D14 - D-3. Die gezeigten Werte sind Mittelwerte aus vier unabhängigen Messungen (n=4).
- **Fig.10:**: Wie Fig. 9, wobei jedoch die Erholung der Hörschwelle am Meerschweinchen nach einem akustisch induzierten Schalltrauma gezeigt wird. Diese berechnet sich aus den Messwerten D0 - D14. Die gezeigten Werte sind Mittelwerte aus vier unabhängigen Messungen (n=4).

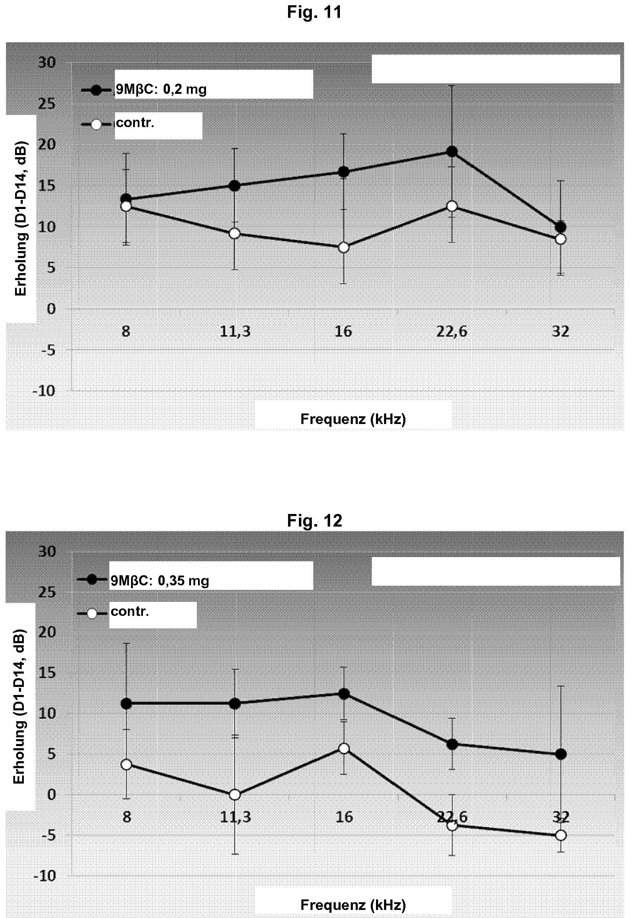

- **Fig. 11**: zeigt die Erholung der Hörschwelle am Meerschweinchen nach einem akustisch induzierten Schalltrauma, wobei ein Ohr nach dem Schalltrauma mit 9-Methyl-β-carbolin (9MβC, insgesamt 0,2 mg pro Ohr) behandelt wurde und das zweite unbehandelte Ohr als intra-individuelle Kontrolle diente (contr.). Die Hörschwelle wurde durch Messung akustisch evozierter Hirnstammpotenziale bestimmt, welche 3 Tage vor dem akustischen Trauma (D-3), nach dem akustischen Trauma (D1) sowie 14 Tage nach dem akustischen Trauma (D14) gemessen wurden. Die Erholung der Hörschwelle berechnet sich aus den Messwerten D1 - D14.
- **Fig. 12:**: Wie Fig. 11, wobei jedoch 0,35 mg 9-Methyl-β-carbolin pro Ohr verabreicht wurden.

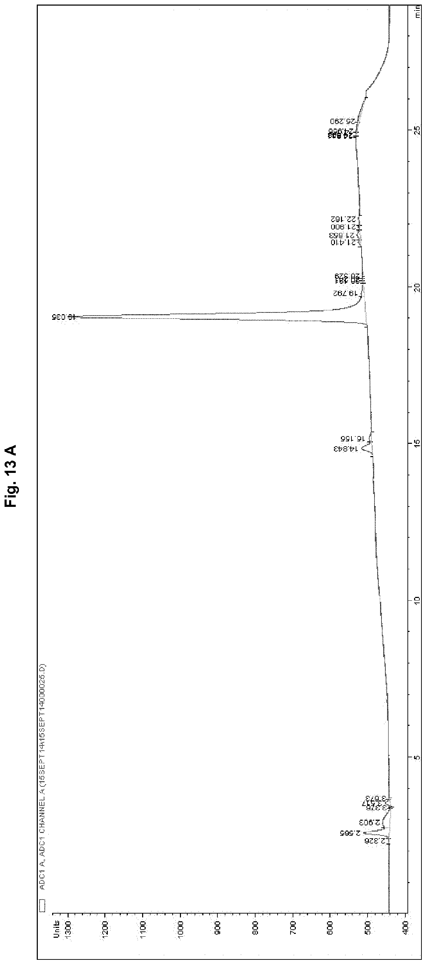

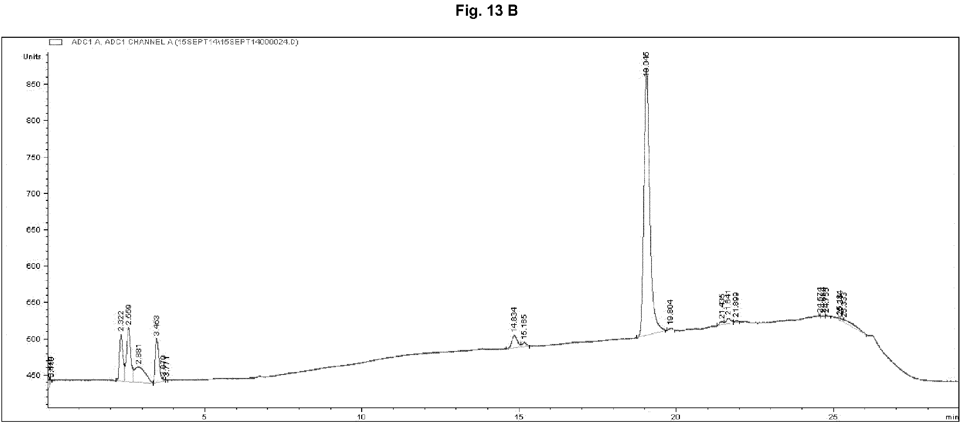

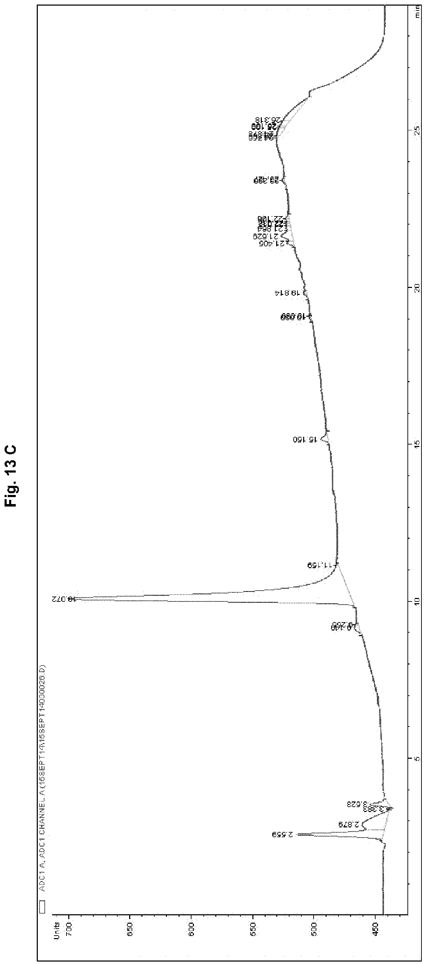

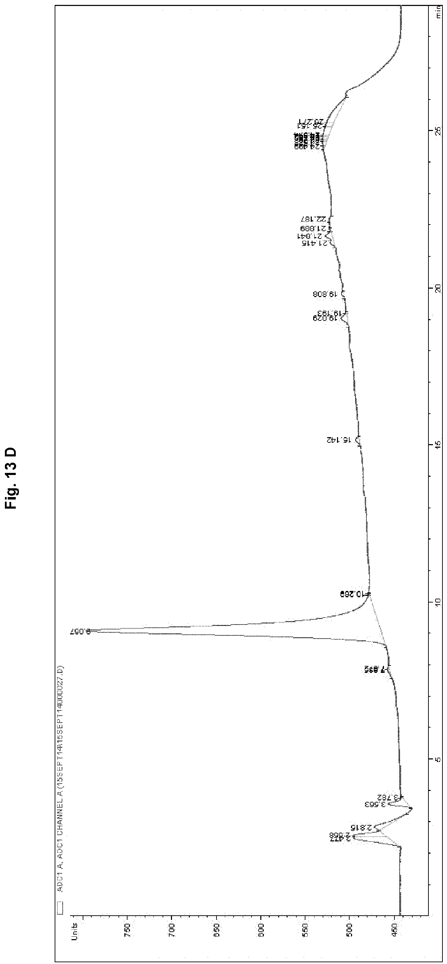

- **Fig. 13:**: HPLC Chromatogramme von unbehandeltem 6-Fluor-9-Methyl-β-carbolin (6F9MβC, **Fig. 13A**), 6-Fluor-9-Methyl-β-carbolin inkubiert für 1 h mit Leberhomogenat (**Fig. 13B**), 6-Fluor-2,9-dimethyl-β-carboliniumion (6F29DMβC, **Fig. 13C**) und 2,9-Dimethyl-β-carboliniumion (29DMβC, **Fig. 13D**). Die Retentionszeiten betrugen 19,0 Min (6F9MβC & Leber + 6F9MβC), 10,1 Min (6F29DMβC) und 9,1 Min (29DMβC). Säule: Phenomenex Luna 3uC18 (2) 100A; Eluent: ACN90%/NH₄Ac 40mM [45/55]

### Beispiele

### Beispiel 1a: Synthese von 6-Fluor-9-methyl-β-carbolin

Analog zur Vorschrift von Ho und Walker (Beng T. Ho und K. E. Walker; Org. Synth. 1971, 51, 136 oder Org. Synth. 1988, Coll. Vol. 6, 965) werden in 10 mL Wasser 3,5 mmol (800 mg) 5-Fluor-1-methyltryptamin Hydrochlorid **(****Figur 1a****, Formel IIa,** AKos GmbH, Steinen) gelöst und mit einer Lösung von 3,9 mmol (356,4 mg) Glyoxalsäurehydrat **(****Figur 1a****, Formel III**) in 810 µL Wasser vereinigt. Unter Rühren wird dann eine Lösung aus 3,4 mmol (190,4 mg) Kaliumhydroxid hinzupipettiert, wobei der pH auf etwa 4 eingestellt wird. Die Reaktionsmischung wird 3 h bei Raumtemperatur gerührt und zur Vervollständigung der Kristallisation noch eine weitere Stunde ins Eisbad gestellt. Der orange-beige Niederschlag des Betains **(****Figur 1a****, Formel IVa)** wird abfiltriert und mit wenig Eiswasser gewaschen.

Zur Decarboxylierung wird der noch feuchte Filterkuchen des Betains **(****Figur 1a****, Formel IVa)** in einen Kolben überführt und in verdünnter Salzsäure (6,48 mLWasser und 918 µL konzentrierte Salzsäure) gelöst. Die Reaktionsmischung wird unter Stickstoff 10 min refluxiert, nochmals 945 µL konzentrierte Salzsäure über ein Septum zugegeben und nochmals 15 min refluxiert. Anschließend wird die Lösung wieder auf Raumtemperatur gebracht und 12 h bei 0-5 °C gelagert. Die entstehenden Kristalle des 6-Fluor-9-methyl-2,3,4,9-tetrahydro-β-carbolins **(****Figur 1a****, Formel Va)** werden abfiltriert und aus Methanol umkristallisiert. Ausbeute: 230 mg (52 %).

Zur Aromatisierung werden die 230 mg 6-Fluor-9-methyl-2,3,4,9-tetrahydro-β-carbolin **(****Figur 1a****, Formel Va)** in 15 mL Wasser gelöst, mit Natriumhydroxid auf pH 13 gebracht und mit einer Mischung aus Ethylacetat und Toluol (1:1) 3 x extrahiert. Die vereinigten organischen Phasen werden mit Magnesiumsulfat (MgSO₄) getrocknet und unter reduziertem Druck eingedampft. Der ölige Rückstand wird mit 50 mL Toluol aufgenommen, mit 100 mg Pd/C (10%) versetzt und 24 h refluxiert. Von der noch warmen Lösung wird Pd/C abfiltriert und der Filterkuchen mit 20 mL warmem Toluol gewaschen. Die organische Phase wird im Vakuum abgedampft. Der ölige Rückstand aus 6-Fluor-9-methyl-β-carbolin **(****Figur 1a****, Formel Ia)** kristallisiert beim Stehen aus und bildet 183 mg (96 %) senfgelbe Kristalle.
Schmelzpunkt: 117-118 °C; GC/MS für die freie Base: m/z = 201 (10 %), 200 (100 %), 199 (71 %), 185 (13 %), 145 (6 %), 131 (6 %), ¹H-NMR: δ (ppm) Methanol *d4,* 400 MHz: 3,80, s, 3H, N-CH₃; 7,34, m, ¹H; 7,43, m, ¹H; 7,73, m, ¹H; 7,91, m, ¹H; 8,24, m, ¹H; 8,73, m, ¹H, ¹³C-NMR: δ (ppm) Methanol d4, 100 MHz: 28,23; 106,38; 106,64; 110,20 and 110,29, d; 114,67; 116,25; 116,52; 120,59 and 120,66, d; 127,91; 131,30; 136,95; 137,51; 138,35; 156,20 and 158,55, d,
Elementaranalyse: für C₁₂H₉FN₂ x 0,1 H₂O x 0,1 Toluol: C, H, N,
Berechnet: C: 72,21; H: 4,77; N: 13,26, Gefunden: C: 72,01; H: 4,55; N: 13,45

### Beispiel 1b: Synthese von 7-Fluor-9-methyl-β-carbolin

Analog zur Synthese von 6-Fluor-9-methyl-β-carbolin werden in 10 mL Wasser 3,5 mmol (800 mg) 6-Fluor-1-methyltryptamin Hydrochlorid **(****Figur 1b****, Formel IIb,** Otavachemicals Ltd., Vaughan, Kanada) gelöst und mit einer Lösung von 3,9 mmol (356,4 mg) Glyoxalsäurehydrat **(****Figur 1b****, Formel III)** in 810 µL Wasser vereinigt. Unter Rühren wird dann eine Lösung aus 3,4 mmol (190,4 mg) Kaliumhydroxid hinzupipettiert, wobei der pH auf etwa 4 eingestellt wird. Die Reaktionsmischung wird 3 h bei Raumtemperatur gerührt und zur Vervollständigung der Kristallisation noch eine weitere Stunde ins Eisbad gestellt. Der orange-beige Niederschlag des Betains **(****Figur 1b****, Formel IVb)** wird abfiltriert und mit wenig Eiswasser gewaschen. Zur Decarboxylierung wird der noch feuchte Filterkuchen des Betains **(****Figur 1b****, Formel IVb)** in einen Kolben überführt und in verdünnter Salzsäure (6,48 mL Wasser und 918 µL konzentrierte Salzsäure) gelöst. Die Reaktionsmischung wird unter Stickstoff 10 min refluxiert, nochmals 945 µL konzentrierte Salzsäure über ein Septum zugegeben und nochmals 15 min refluxiert. Anschließend wird die Lösung wieder auf Raumtemperatur gebracht und 12 h bei 0-5 °C gelagert, wobei 7-Fluor-9-methyl-2,3,4,9-tetrahydro-β-carbolin als HCl-Salz **(****Figur 1b****, Formel Vb)** ausfällt. Die entstehenden Kristalle werden abfiltriert und aus Methanol umkristallisiert. Ausbeute: 1 mmol bzw. 250 mg (28 %).

Zur Aromatisierung werden die 250 mg 7-Fluor-9-methyl-2,3,4,9-tetrahydro-β-carbolin x HCl **(****Figur 1b****, Formel Vb)** in 15 mL Wasser gelöst, mit verd. Kaliumhydroxid Lösung auf pH 13 gebracht und über Nacht im Kühlschrank gelagert. Die freie Base kristallisiert dabei als beiger, kristalliner Feststoff aus, der abfiltriert, mit Wasser gewaschen und im Vakuum getrocknet wird. Der Feststoff wird in 50 mL Toluol suspendiert, mit 100 mg Pd/C (10%) versetzt und 8 h refluxiert (DC als Umsatzkontrolle). Von der noch warmen Lösung wird Pd/C abfiltriert und der Filterkuchen mit 20 mL warmem Toluol gewaschen. Die organische Phase wird im Vakuum abgedampft. Der ölige Rückstand aus 7-Fluor-9-methyl-β-carbolin **(****Figur 1b****, Formel Ib)** kristallisiert beim Stehen aus und bildet senfgelbe Kristalle, die säulenchromatographisch (CHCl₃ / Methanol 9:1) gereinigt werden. Es verbleiben 160 mg (80 %).

### Beispiel 2: Differenzierung von humanen Neuroblastomzellen (SH-SY5Y)

Die SH-SY5Y-Zelllinie wurde von der deutschen Sammlung von Mikroorganismen und Zellkulturen - DSMZ, Braunschweig bezogen. Die Zellen wurden in MEM (Minimum essential medium, Life Technologies) mit 10 % FCS (fetales Kälberserum, Biochrom AG) und Penicillin/Streptomycin (Biochrom AG) kultiviert. Für die morphologischen Untersuchungen wurden die Zellen in einer Dichte von 1,5 x 10⁴ Zellen/cm² in 24-Wellplatten (Falcon, cell culture treated) mit 1 mL Medium ausgesät. Eine Stammlösung von 10 mmol/L der erfindungsgemäßen Verbindung 6-Fluor-9-methyl-β-carbolin wurde in 50 % Ethanol hergestellt und steril filtriert. Fünf Stunden nach dem Aussäen der Zellen wurde die erfindungsgemäße Verbindung 6-Fluor-9-methyl-β-carbolin in der entsprechenden Menge zum Medium gegeben, um die gewünschte Endkonzentration zu erhalten. Das Medium wurde einmal pro Woche gewechselt. Die Zellen wurden regelmäßig mikroskopisch begutachtet. Mikroskopische Aufnahmen wurden am BZ Keyence 10 mit einem 10 x Objektiv und 4 x Digitalzoom aufgenommen. Eine automatische Kontrastverstärkung wurde mit dem Programm BZ Analyzer vorgenommen.

Die Befunde ergeben einen Vorteil der erfindungsgemäßen Verbindung 6-Fluor-9-methyl-β-carbolin gegenüber 9-Methyl-β-carbolin, da eine vergleichbare Differenzierung der getesteten Zellen erst bei einer Konzentration von etwa 70 µM 9-Methyl-β-carbolin im Vergleich zu 30 µM 6-Fluor-9-Methyl-β-carbolin beobachtet wird. 50 µM 9-Methyl-β-carbolin führten zu einem geringeren Neuritenwachstum, als Anzeichen einer Differenzierung der humanen Neuroblastomzellen, als 30 µM der erfindungsgemäßen Verbindung 6-Fluor-9-methyl-β-carbolin.

### Beispiel 3: Lipophilie von 6-Fluor-9-methyl-β-carbolin im Vergleich zu 9-Methyl-β-carbolin

Die folgenden Experimente wurden durchgeführt um zu prüfen, ob die Lipophilie von 6-Fluor-9-methyl-β-carbolin größer ist als die des 9-Methyl-β-carbolins. Experimentell wird die Lipophilie entweder durch die tatsächliche Verteilung zwischen Octanol und Wasser bestimmt, oder einfacher, wie von Valkó beschrieben, durch HPLC-Experimente mit einer Umkehrphasen-Säule (RP18) (K. Valkó, Journal of Chromatography A 2004,1037 (1-2), 299-310). Hierbei wechselwirken die Bestandteile der zu untersuchenden Substanz unterschiedlich stark mit der stationären Phase. Wechselwirkt ein Bestandteil schwächer mit der stationären Phase, verlässt er die Säule früher. Je nach Stärke dieser Wechselwirkungen erscheinen die Bestandteile der Substanz zu verschiedenen Zeiten (den Retentionszeiten) am Ende der Trennsäule. Solche Experimente wurden für die beiden Substanzen durchgeführt. Es zeigte sich, dass die erfindungsgemäße Verbindung 6-Fluor-9-methyl-β-carbolin eine längere Retentionszeit hat als 9-Methyl-β-carbolin (Figur 4), was bei der ausgewählten Säule bzw. stationären Phase eindeutig auf eine höhere Lipophilie des 6-Fluor-9-methyl-β-carbolins hinweist.

Zudem wurde die Löslichkeit beider Verbindungen in Wasser bestimmt, indem für beide Verbindungen eine Konzentrationsreihe in Wasser bei Raumtemperatur angesetzt wurde. Die Konzentration, die noch gerade löslich war, wurde durch eine externe Kalibrierung bestimmt. Die entsprechende Konzentration wurde mittels HPLC quantifiziert. Es wurde eine maximal lösliche Konzentration des 6-Fluor-9-methyl-β-carbolins (Base) von 29,7 mg/L und des 9-Methyl-β-carbolins (Base) von 168,3 mg/L gefunden. Diese geringere Wasserlöslichkeit des 6-Fluor-9-methyl-β-carbolins stützt im Umkehrschluss die erhöhte Lipophilie im Vergleich zu 9-Methyl-β-carbolin.

Die Lipophilie eines Stoffes hat einen starken Effekt auf seine ADME Parameter ("absorption", "distribution", "metabolism", und "excretion"). Durch die gesteigerte Lipophilie werden Stoffe vor allem in lipophilen Kompartimenten angereichert. Um Membranen passieren zu können, müssen chemische Stoffe den hydrophoben Bereich einer biologischen Doppelmembran passieren können, sie müssen also selbst lipophil sein. Oft besteht eine gute Korrelation zwischen Lipidlöslichkeit und Membranpassage. Somit beeinflusst die Lipophilie ganz entscheidend die Pharmakokinetik einer Verbindung. Zusammengefasst zeigen mehrere Experimente eine deutliche höhere Lipophilie von 6-Fluor-9-methyl-β-carbolin gegenüber 9-Methyl-β-carbolin

Ein weiterer wichtiger Aspekt der erfindungsgemäßen Verbindung betrifft die Verlängerung der Eliminationshalbwertszeit. Während das 9-Methyl-β-carbolin über eine Hydroxylierung an Position 6 mit anschließender Konjugationsreaktion abgebaut wird, ist dieser Metabolisierungsweg beim 6-Fluor-9-methyl-β-carbolin durch die Fluoridgruppe blockiert. Wahrscheinlich ist dies der Grund für eine Erhöhung der Halbwertszeit des 6-Fluor-9-methyl-β-carbolins verglichen mit 9-Methyl-β-carbolin. Da die Halbwertszeit von Harman, einem Strukturanalogon von 9-Methyl-β-carbolin, im Blutplasma des Menschen etwa 68 Min beträgt (Rommelspacher et al.; Eu J Pharmacol 2002, 441 (1-2), 115-125), stellt eine Verlängerung dieses Werts einen relevanten Vorteil der erfindungsgemäßen Verbindung dar, insbesondere bei Anwendungen zur Behandlung von chronischen Erkrankungen.

Zur besseren Beurteilung der Halbwertzeiten von 9-Methyl-β-carbolin (9MβC), 6-Fluor-9-methyl-β-carbolin (6F9MβC) und 7-Fluor-9-methyl-β-carbolin (7F9MβC) im tierischen Organismus bzw. genauer in der Perilymphe (dem avisierten Wirkungsort) wurden Experimente an Meerschweinchen durchgeführt. Hierzu wurde narkotisierten Meerschweinchen der jeweilige Wirkstoff (insgesamt jeweils 1,4 mg Wirkstoff pro Ohr), verpackt in 40 µl eines Hydrogels (18% Poloxamer 407 und 0,6% Alginat), einmalig vor die Membran des runden Fensters im Mittelohr appliziert. Diese Membran trennt das mit Luft gefüllte Mittelohr von dem mit der Perilymphe gefüllten Innenohr. Nach Ablauf der gewünschten Zeit (20 min, 2 h, 8 h bzw. 24 h) wurden unter Narkose jeweils 5µL Perilymphe aus der Spitze der Hörschnecke (Apex) abgezogen. Anschließend wurde das Tier getötet. Pro Zeit (20 min, 2 h, 8 h bzw. 24 h) wurden Messwerte aus zumindest drei Ohren genommen und gemittelt.

Die Konzentration des jeweiligen Wirkstoffes in den Perilymph-Proben wurde über Hochleistungsflüssigkeitschromatographie (HPLC, z.T. auch als Hochdruckflüssigkeitschromatographie bezeichnet) und Messung der Eigen-Fluoreszenz des jeweiligen Wirkstoffes bestimmt. Bei der HPLC bestand der Eluent zu 45 % aus 90%igem Acetonitril und zu 55 % aus Ammoniumazetat (40 mM, pH <5). Die Flussrate betrug 0,4 mL/min und das Injektionsvolumen war 10 µL. Jede Probe wurde mit 60 % Methanol verdünnt, sofern das Signal zu stark war. Nach jedem Lauf wurde die Injektionsnadel mit 100 % Isopropanol gespült. Die Nachweisgrenze betrug 20 fmol/ L.

Aus den ermittelten Wirkstoffkonzentrationen konnten dann die Eliminationshalbwertszeiten errechnet werden. Es zeigte sich hierbei für das 9-Methyl-β-carbolin eine Eliminationshalbwertszeit von cirka 3,4 h, für das 6-Fluor-9-methyl-β-carbolin eine Eliminationshalbwertszeit von cirka 6,0 h und für das 7-Fluor-9-methyl-β-carbolin eine Eliminationshalbwertszeit von cirka 5,0 h. Durch die 1,5fache (7F9MβC) bis 1,75fache Steigerung (6F9MβC) der Eliminationshalbwertszeit der erfindungsgemäßen Substanzen gegenüber dem 9-Methyl-β-carbolin ergibt sich eine deutlich verbesserte Bioverfügbarkeit im Vergleich zum Stand der Technik. Insgesamt fällt auf, dass die in der Perilymphe ermittelten Halbwertzeiten höher sind als die für das Blut geschätzten Halbwertzeiten (z.B. in Bezug zum 9MβC).

### Beispiel 4: quantitative RT-PCR-Experimente I

An Neuroblastomzellen sollte untersucht werden, inwieweit die erfindungsgemäßen Verbindungen, 6-Fluor-9-Methyl-β-carbolin und 7-Fluor-9-Methyl-β-carbolin, sowie das 9-Methyl-β-carbolin aus dem Stand der Technik auf die Expression wichtiger neuroprotektiver bzw. neurotropher Faktoren wirkt, von denen eine regenerierende Wirkung auf geschädigte Hörsinneszellen bekannt ist. Außerdem verbessern sie die Vitalität der Nervenzellen des Spiralganglions, die die Information aus dem Innenohr über den Hirnstamm und weiter ins Hörzentrum des Gehirns vermitteln. Bei einem Trauma ziehen sich die Nervenfortsätze von den Haarsinneszellen zurück, wodurch das Signal der entsprechenden Töne nicht mehr weitergeleitet wird. Die neurotrophen Faktoren bewirken, dass die Verbindung zwischen den Nervenfortsätzen und den passenden Haarsinneszellen wieder geknüpft sodass die entsprechenden Töne wieder gehört werden.

Für die hierzu durchgeführten quantitativen RT-PCR Experimente (Reverse Transkriptase Polymerase Kettenreaktion) wurden erneut SH-SY5Y-Zelllinien verwendet, welche vom Institut für Humangenetik des Universitätsklinikums Eppendorf zur Verfügung gestellt wurden, die wiederum die Zellen vom Braunschweig Ressourcenzentrum bezogen hatten.

Die Zellen wurden in MEM (Minimum essential medium, Life Technologies) mit 10% FCS (fetales Kälberserum, Biochrom AG) und Penicillin/Streptomycin (Biochrom AG) kultiviert. Die Zellen wurden in einer Dichte von 1x10⁵ Zellen/cm² in 25 cm² Zellkulturflaschen (Falcon, cell culture treated) mit 5 ml Medium ausgesät. Stammlösungen von 10 mmol/L der Substanzen 9-Methyl-β-carbolin (AC-002 bzw. 9MβC) bzw. 6-Fluor-9-methyl-β-carbolin (AC102 bzw. 6F9MβC) wurden in destilliertem Wasser hergestellt und steril filtriert. Einen Tag nach dem Aussäen der Zellen wurden die Substanzen in den entsprechenden Mengen hinzugegeben (5-55 µl) um finale Konzentration im Medium von 10 µM, 20 µM und 30 µM für 6F9MβC und 30 µM, 50 µM und 70 µM für 9MβC zu erhalten. Die Kontrollen erhielten lediglich die entsprechende Menge an Lösungsmittel (destilliertes Wasser). Das Medium wurde ein Mal pro Woche gewechselt.

Die Zellen wurden regelmäßig mikroskopisch begutachtet und nach 2, 7 und 14 Tagen Inkubationsdauer geerntet, mit PBS gewaschen und sofort bei -80°C eingefroren.

Die RNA-Isolierung erfolgte mit dem HighPure RNA Isolation Kit der Firma Roche. Diese Isolierung beinhaltet einen DNasel-Verdau. Die Konzentration der RNA wurde am nanoDrop Spektrometer gemessen und die RNA-Integrität wurde mit Hilfe des Bioanalyzers 2100 (Agilent) bestimmt. Pro Probe wurden, unter Verwendung von Random-Hexamerprimern, des Protector RNA inhibitors (Roche) und der Transcriptor Reverse Transcriptase (Fermentas), 300 ng RNA in cDNA umgeschrieben.

Nach der Reaktion wurde dieser Ansatz 1:4 verdünnt und davon wurden jeweils 2 µl pro quantitativer RT-PCR eingesetzt. Diese wurde im LightCycler 480 unter Standardbedingungen im SybrGreen-Format mit den für die jeweiligen Primer ermittelten Annealingtemperaturen durchgeführt. Falls möglich, wurden exonüberspannende Primer verwendet.

Die Liste der verwendeten Primer findet sich in Tabelle 1.

**Tab. 1: Primer-Sequenzen für die quantitativen RT-PCR Experimente. In Klammern sind zudem die jeweiligen Sequenz-ID-Nummern (SEQ-ID) aufgeführt. Die Sequenz aller Primer ist in 5'-3'-Richtung angegeben.**

| **Zielgen** | **Forward-Primer** | **Reverse-Primer** |
|---|---|---|
| BDNF (*brain*-*derived neurotrophic factor)* | GGATGAGGACCAGAAAGT (SEQ-ID 01) | AGCAGAAAGAGAAGAGGAG (SEQ-ID 02) |
| Lif (*Leukemia inhibitory factor)* | CCAACAACCTGGACAAGCTATG (SEQ-ID 03) | GTGGCGTTGAGCTTGCTGTG (SEQ-ID 04) |
| NT3 (*Neurotrophin 3)* | CGGAGCATAAGAGTCACC (SEQ-ID 05) | CCTGGCTTCCTTACATCG (SEQ-ID 06) |
| Id2 (*Inhibitor of Differentiation 2)* | CATCCTGTCCTTGCAGGCTT (SEQ-ID 07) | CCATTCAACTTGTCCTCCTTGTG (SEQ-ID 08) |
| BTG2 (*B-cell translocation gene 2)* | CAGGAGGCACTCACAGAGCA (SEQ-ID 09) | AATGCGGTAGGACACCTCATA (SEQ-ID 10) |
| Cbln (*Cerebellin 1 precursor Protein)* | CAAGTGCCTGGTGGTGT (SEQ-ID 11) | GTTCACTAGTACCTGGTCGAAGTAG (SEQ-ID 12) |
| DRD2S (*kurze Variante des Dopaminrezeptors Subtyp 2)* | GGACTCAATAACGCAGACCAGAA (SEQ-ID 13) | CGGGCAGCCTCCTTTAGT (SEQ-ID 14) |
| DKK1 (*Dickkopf-related Protein 1)* | CATTGACAACTACCAGCCGT (SEQ-ID 15) | ATCAGAAGACACACATATTCCATT (SEQ-ID 16) |
| Bax | GATGATTGCCGCCGTGGACA (SEQ-ID 17) | CACCTTGGTGCACAGGGCCTT (SEQ-ID 18) |
| Bcl2 | GTGTGGAGAGCGTCAACC (SEQ-ID 19) | CTTCAGAGACAGCCAGGAG (SEQ-ID 20) |
| B2M (*β2-Mikroglobulin)* | ACTGGTCTTTCTATCTCTTGTACT (SEQ-ID 21) | CTTCAAACCTCCATGATGCT (SEQ-ID 22) |

Die Spezifität wurde über Schmelzkurvenanalyse und nachträgliche Gelelektrophorese überprüft. Für jede PCR wurden über das Programm LinRegPCR die PCR-Effizienz und der Cq-Wert bestimmt und diese in den weiteren Berechnungen verwendet. Aus mehreren Referenzgenen wurde B2M (β2-Mikroglobulin) als geeignet für die Untersuchungsbedingungen ermittelt. Alle Ergebnisse sind relative Ergebnisse zu den jeweiligen Kontrollbedingungen, bei denen für die angegebene Zeitdauer (d.h. 2 Tage, 7 Tage oder 14 Tage) lediglich Lösungsmittel verabreicht wurde. In den Figuren 5A bis 5I wird also die relative Genexpression (relativ zu den Kontrollbedingungen) des jeweiligen Faktors dargestellt. Hierbei wird die 2-tägige Exposition in hellgrau, die 7-tägige Exposition in dunkelgrau und die 14-tägige Exposition in schwarz dargestellt. Jeder Messwert aus den Figuren 5A - 5I stellt einen Mittelwert aus 3 unabhängigen biologischen Proben dar.

Figur 5A zeigt die relative Genexpression des Neurotrophins BDNF (*brain*-*derived neurotrophic factor*) nach 2 Tagen, 7 Tagen und 14 Tagen Exposition gegenüber 6F9MβC (10 µM, 20 µM & 30 µM) oder 9MβC (30 µM, 50 µM & 70 µM). Vergleicht man die BDNF-Expression nach Exposition mit 30 µM 6F9MβC oder 9MβC, so zeigt sich bereits bei dieser Konzentration, dass 6F9MβC zu einer ca. doppelt so hohen Expression von BDNF führt wie das 9MβC.

Figur 5B zeigt die relative Genexpression von Lif (*Leukemia inhibitory factor*), welcher die Zelldifferenzierung stimuliert und die Proliferation hemmt, nach 2 Tagen, 7 Tagen und 14 Tagen Exposition gegenüber 6F9MβC (10 µM, 20 µM & 30 µM) oder 9MβC (30 µM, 50 µM & 70 µM). Vergleicht man die Lif-Expression nach Exposition mit 30 µM 6F9MβC oder 9MβC, so zeigt sich bereits bei dieser Konzentration, dass 6F9MβC nach nur 2-tägiger Exposition zu einer ca. dreifach so hohen Expression von Lif führt wie das 9MβC. Auch nach 14-tägiger Exposition gegenüber 6F9MβC ist die Lif-Expression ca. doppelt so hoch wie im Vergleich zu 9MβC. Lediglich nach 7-tägiger Exposition gegenüber 6F9MβC scheint die Expression von Lif etwas geringer als im Vergleich zu 9MβC. Bemerkenswert ist zudem die hohe Expression bei geringeren Konzentrationen an 6F9MβC.

Figur 5C zeigt die relative Genexpression des Neurotrophins NT3 (*Neurotrophin 3*) nach 2 Tagen, 7 Tagen und 14 Tagen Exposition gegenüber 6F9MβC (10 µM, 20 µM & 30 µM) oder 9MβC (30 µM, 50 µM & 70 µM). Vergleicht man die NT3-Expression nach Exposition mit 30 µM 6F9MβC oder 9MβC, so zeigt sich bei dieser Konzentration, dass 6F9MβC nach 14-tägiger Exposition zu einer ca. 3,5fach höheren Expression von NT3 führt wie das 9MβC. Bemerkenswert ist zudem die hohe Expression von NT3 bei geringeren Konzentrationen an 6F9MβC, besonders nach 7-tägier Exposition mit lediglich 10 µM 6F9MβC.

Figur 5D zeigt die relative Genexpression von Id2 (*Inhibitor of Differentiation 2*), welcher die Zelldifferenzierung hemmt und so die Proliferation begünstigt. Es wurde auch beschrieben, dass Mäuse, bei denen das Gen ausgeschaltet worden war, neurodegenerative Erscheinungen zeigten. Gezeigt wird die relative Genexpression von Id2 nach 2 Tagen, 7 Tagen und 14 Tagen Exposition gegenüber 6F9MβC (10 µM, 20 µM & 30 µM) oder 9MβC (30 µM, 50 µM & 70 µM). Vergleicht man die Id2-Expression nach Exposition mit 30 µM 6F9MC oder 9MβC, so zeigt sich bereits bei dieser Konzentration, dass 6F9MβC zu einer ca. 2-fach bis 3,5-fach höheren Expression von Id2 führt wie das 9MβC. Bemerkenswert ist auch hier die hohe Expression von Id2 bei geringeren Konzentrationen an 6F9MβC.

Figur 5E zeigt die relative Genexpression des Neuroprotektors BTG2 (*B-cell translocation gene 2*) nach 2 Tagen, 7 Tagen und 14 Tagen Exposition gegenüber 6F9MβC (10 µM, 20 µM & 30 µM) oder 9MβC (30 µM, 50 µM & 70 µM). Vergleicht man die BTG2-Expression nach Exposition mit 30 µM 6F9MβC oder 9MβC, so zeigt sich bereits bei dieser Konzentration, dass 6F9MβC zu einer ca. 1,25-fach bis 2,7-fach höheren Expression von BTG2 führt wie das 9MβC. Bemerkenswert ist zudem, dass bereits bei 10 µM 6F9MβC eine vergleichbare Expression von BTG2 zu verzeichnen ist wie bei der höchsten Konzentration an 9MβC (ausgenommen nach 2-tägiger Exposition).

Figur 5F zeigt die relative Genexpression des Neuroprotektors Cbln (*Cerebellin 1 precursor Protein*) nach 2 Tagen, 7 Tagen und 14 Tagen Exposition gegenüber 6F9MβC (10 µM, 20 µM & 30 µM) oder 9MβC (30 µM, 50 µM & 70 µM). Vergleicht man die Cbln-Expression nach Exposition mit 30 µM 6F9MβC oder 9MβC, so zeigt sich bei dieser Konzentration, dass 6F9MβC zu einer ca. 1,5-fach höheren Expression von Cbln führt wie das 9MβC (ausgenommen nach 2-tägiger Exposition). Bei lediglich 20 µM 6F9MβC scheint die Expression von Cbln sogar noch höher zu sein.

Figur 5G zeigt den Quotienten der Genexpression von Bax und Bcl2, welcher als Apoptose-Marker zählt, nach 2 Tagen, 7 Tagen und 14 Tagen Exposition gegenüber 6F9MβC (10 µM, 20 µM & 30 µM) oder 9MβC (30 µM, 50 µM & 70 µM). Je geringer der Quotient Bax/Bcl2 umso weniger Apoptose. Vergleicht man den Quotienten Bax/Bcl2 nach Exposition mit 30 µM 6F9MβC oder 9MβC, so ist dieser nach 6F9MβC deutlich geringer als nach 9MβC (ausgenommen nach 2-tägiger Exposition). Bemerkenswert ist zudem der niedrige Quotient Bax/Bcl2 bei geringeren Konzentrationen an 6F9MβC.

Figur 5H zeigt die relative Genexpression von DRD2S (*kurze Variante des Dopaminrezeptors Subtyp 2*) nach 2 Tagen, 7 Tagen und 14 Tagen Exposition gegenüber 6F9MβC (10 µM, 20 µM & 30 µM) oder 9MβC (30 µM, 50 µM & 70 µM). Vergleicht man die DRD2S-Expression nach Exposition mit 30 µM 6F9MβC oder 9MβC, so zeigt sich bei dieser Konzentration, dass 6F9MβC bei 2-tägiger und 14-tägiger Exposition zu einer höheren Expression von DRD2S führt wie das 9MβC und nach 7-tägiger Exposition zu einer geringeren Expression von DRD2S führt wie das 9MβC. Bei geringeren Konzentrationen an 6F9MβC scheint die Expression von DRD2S nach 7-tägiger Expression vergleichbar mit der nach 9MβC.

Figur 5I zeigt die relative Genexpression des Faktors DKK1 (*Dickkopf-related Protein* 1), der für die Differenzierung von Nervenzellen von Bedeutung ist und nach aktuelle Erkenntnissen auch eine Rolle bei Traumata von Sinneszellen spielt, nach 2 Tagen, 7 Tagen und 14 Tagen Exposition gegenüber 6F9MβC (10 µM, 20 µM & 30 µM) oder 9MβC (30 µM, 50 µM & 70 µM). Vergleicht man die DKK1-Expression nach Exposition mit 30 µM 6F9MβC oder 9MβC, so zeigt sich gerade bei kürzene Expositionen (2 Tage und 7 Tage), dass 6F9MβC zu einer ca. 1,4-fach bis 2,7-fach höheren Expression von DKK1 führt wie das 9MβC. Bei 14-tägiger Exposition sind die Werte vergleichbar. Bemerkenswert ist zudem, dass bereits bei 10 µM 6F9MβC eine vergleichbare wenn nicht gar höhere Expression von DKK1 zu verzeichnen ist als bei der höchsten Konzentration an 9MβC.

Insgesamt zeigt sich also, dass das erfindungsgemäße 6-Fluor-9-methyl-β-carbolin in praktisch allen untersuchten zellulären Faktoren, welche direkt oder indirekt einen Einfluss auf die Differenzierung und Proliferation von Zellen und damit auch auf eine Regeneration von Sinnesgeweben nach Traumata haben, einen vorteilhaften Effekt gegenüber dem 9-Methyl-β-carbolin aus dem Stand der Technik hat. Besonders hervorzuheben sind hier die Faktoren BDNF und Lif, die zu den wichtigsten Faktoren für zelluläre Reparaturvorgänge nach Traumata im Innenohr zählen. Das erfindungsgemäße 6-Fluor-9-methyl-β-carbolin hat somit das Potential nicht nur erfolgreich bei der Behandlung von Erkrankungen und/oder Schädigungen des Innenohres eingesetzt zu werden, sondern zudem auch in einer niedrigeren Konzentration als die Methyl-β-carboline aus dem Stand der Technik.

### Beispiel 5: quantitative RT-PCR-Experimente II

Analog zu Beispiel 4 wurden für einige wichtige zelluläre Faktoren ebenfalls RT-PCR-Experimente mit dem erfindungsgemäßen Wirkstoff 7-Fluor-9-methyl-β-carbolin (7F9MβC) durchgeführt. Die Versuchsdurchführung hierbei war identisch zu den Versuchen mit 6-Fluor-9-methyl-β-carbolin und 9-Methyl-β-carbolin. Auch hier wurden (wie für das 6F9MβC) finale Konzentrationen von 30 µM, 50 µM und 70 µM 7F9MβC im Kulturmedium eingesetzt. Bei den untersuchten Faktoren handelte es sich um BDNF (*Brain-derived neurotrophic Factor*), Lif (*Leukemia inhibitory factor*), DRD2S (kurze Variante des Dopaminrezeptors Subtyp 2) sowie DKK1 (*Dickkopf-related Protein 1*). Es wurden die gleichen Primer verwendet, die bereits für die entsprechenden Zielgene in Beispiel 4 (Tab. 1) aufgelistet sind. Wie bereits erwähnt, zählen besonders BDNF und Lif zu den wichtigsten Faktoren für zelluläre Reparaturvorgänge nach Traumata im Innenohr.

Figur 6A zeigt die relative Genexpression des Neurotrophins BDNF (*brain*-*derived neurotrophic factor*) nach 2 Tagen, 7 Tagen und 14 Tagen Exposition gegenüber 7F9MβC (10 µM, 20 µM & 30 µM). Vergleicht man die BDNF-Expression nach Exposition mit 30 µM 7F9MβC und mit 30 µM 9MβC (Fig. 5A), so zeigt sich bei einer 2-tägigen Exposition, dass 7F9MβC zu einer dreifach höheren Expression von BDNF führt wie das 9MβC. Bei längeren Expositionen sind die Werte vergleichbar.

Figur 6B zeigt die relative Genexpression von Lif (*Leukemia inhibitory factor*), welcher die Zelldifferenzierung stimuliert und die Proliferation hemmt, nach 2 Tagen, 7 Tagen und 14 Tagen Exposition gegenüber 7F9MβC (10 µM, 20 µM & 30 µM). Vergleicht man die Lif-Expression nach Exposition mit 30 µM 7F9MβC und mit 30 µM 9MβC (Fig. 5B), so zeigt sich bei einer 2-tägigen Exposition, dass 7F9MβC zu einer dreifach höheren Expression von Lif führt wie das 9MβC. Bei 7-tägiger Exposition ist die Induktion durch 7F9MβC etwas schwächer als durch 9MβC und bei 14-tägiger Exposition sind die Werte vergleichbar. Bemerkenswert ist zudem die hohe Expression von Lif nach 2-tägiger Exposition mit 20 µM 7F9MβC.

Figur 6C zeigt die relative Genexpression von DRD2S (*kurze Variante des Dopaminrezeptors Subtyp* 2) nach 2 Tagen, 7 Tagen und 14 Tagen Exposition gegenüber 7F9MβC (10 µM, 20 µM & 30 µM). Vergleicht man die DRD2S-Expression nach Exposition mit 30 µM 7F9MβC und mit 30 µM 9MβC (Fig. 5H), so zeigt sich bei einer 2-tägigen Exposition, dass 7F9MβC zu einer zweifach höheren Expression von DRD2S führt wie das 9MβC. Bei 7-tägiger Exposition sind die Werte vergleichbar und bei 14-tägiger Exposition ist die Induktion durch 7F9MβC etwas schwächer als durch 9MβC.

Figur 6D zeigt die relative Genexpression des Faktors DKK1 (*Dickkopf*-*related Protein 1*), der für die Differenzierung von Nervenzellen von Bedeutung ist und nach aktuelle Erkenntnissen auch eine Rolle bei Traumata von Sinneszellen spielt, nach 2 Tagen, 7 Tagen und 14 Tagen Exposition gegenüber 7F9MβC (10 µM, 20 µM & 30 µM). Vergleicht man die DKK1-Expression nach Exposition mit 30 µM 7F9MβC und mit 30 µM 9MβC (Fig. 5I), so zeigt sich bei einer 2-tägigen Exposition, dass 7F9MβC zu einer cirka 1,4-fach höheren Expression von DKK1 führt wie das 9MβC. Bei 7-tägiger Exposition sind die Werte vergleichbar und bei 14-tägiger Exposition ist die Induktion durch 7F9MβC cirka 2,2-fach höher als durch 9MβC.

Insgesamt fällt auf, dass das 7-Fluor-9-methyl-β-carbolin im Vergleich zum 9-Methyl-β-carbolin aus dem Stand der Technik gerade bei einer kurzen Exposition höhere Effekte erzielt. Ein möglicher Erklärungsansatz ist, dass das 7-Fluor-9-methyl-β-carbolin lipophiler ist als das 9-Methyl-β-carbolin und deshalb schneller in Zellen eindringt und somit schneller höhere Konzentrationen in der Zelle erreicht. Aus therapeutischer Sicht sind derartige Vorteile natürlich wünschenswert, da sie eine effiziente und kürzere Behandlungsdauer ermöglichen, was dem Wohl des Patienten dient. Vom therapeutischen Standpunkt aus ist das 7-Fluor-9-methyl-β-carbolin deshalb zur Behandlung von akuten Schäden des Innenohrs wie z.B. Knalltrauma und Hörsturz dem 9-Methyl-β-carbolin aus dem Stand der Technik überlegen.

### Beispiel 6: Immunhistochemische Experimente

Im Folgenden sollte auf histologischer Basis am Meerschweinchen untersucht werden, wie sich die Verabreichung von 9-Methyl-β-carbolin (9MβC) bzw. von 6-Fluor-9-methyl-β-carbolin (6F9MβC) auf das durch ein Knalltrauma induzierte Absterben der äußeren Haarzellen (*outer hair cells*, OHC) im Innenohr auswirkt auswirkt. Es soll an dieser Stelle erwähnt werden, dass die inneren Haarzellen (IHC) gegenüber jeglichem Trauma unempfindlicher sind.

Um ein akustisches Traum auszulösen wurden Meerschweinchen mit 60mg/kg Ketamin und 8mg/kg Xylazin betäubt. Dann wurde Bepantensalbe (Bayer) auf die Augen appliziert und das Tier in eine schalldichte Kammer gelegt. Ein kalibrierter Lautsprecher wurde direkt über dem Kopf der Tiers angebracht, wobei dieser so eingestellt war, dass er ein Halboktavband, um 8kHz zentriert, mit einer Lautstärke von 110 dB über eine Dauer von 30 Minuten emittierte.

Das 6-Fluor-9-methyl-β-carbolin bzw. 9-Methyl-β-carbolin, verpackt in ein Hydrogel (18% Poloxamer 407 und 0,6% Alginat), wurde für jeden Versuch frisch zubereitet und bis zur Anwendung bei +4°C im Kühlschrank aufbewahrt.

Das thermosensitive, flüssige Gemisch wurde unter Sicht mittels einer Hamilton Spritze direkt vor die Rundfenstermembran des Mittelohrs appliziert (10 µL). Die erste Dosis 6-Fluor-9-methyl-β-carbolin (z.B. 0,07mg) wurde eine Stunde nach dem Schalltrauma appliziert (Tag 0) bei einer Konzentration von 0.007 mg/µl und die zweite Dosis 6-Fluor-9-methyl-β-carbolin (0,035mg in einem Volumen von 5 µL) am Tag 3. Hierbei wurde jeweils nur ein Ohr pro Tier behandelt, während das zweite Ohr als intra-individuelle Kontrolle diente. Die Kontrolle wird hierin zum Teil auch mit "contr." abgekürzt. In Vergleichsexperimenten wurde festgestellt, dass eine bloße Gabe des Wirkstoff-Vehikels (also des Hydrogels) im Vergleich zu einem vollkommen unbehandelten Ohr keinen Effekt hatte. Für jeden Wirkstoff (inklusive der Kontrollen) wurden jeweils vier Meerschweinchen untersucht.

Am Tag 14 nach dem Schalltrauma wurde die Region des Ohrs präpariert, sodass diese die Cochlea enthielt. Das Präparat wurde in eiskaltes 4% Paraformaldehyd (PFA) für 24 Stunden eingelegt und bei +4°C aufbewahrt. Nach der PFA-Fixierung wurde das Präparat 2 mal 5 Minuten mit Phosphat gepufferter Kochsalzlösung (PBS) gespült und danach in eine 10% Ethylendiamintetraessigsäure (EDTA) zur Dekalzifizierung gelegt. Nach mindestens 48 Stunden wurde das Präparat zweimal mit PBS gespült und dann die Cochlea herauspräpariert. Das gesamte Cortische Organ, das die Sinneszellen enthält, wurde dann aus der Cochlea herauspräpariert und in vier gleich lange Abschnitte geschnitten. Jeder der Abschnitte entspricht also dem Cortischen Organ von ¾ einer Cochleawindung. Auf diese Weise kann der schallbedingte Schaden lokalisiert und quantifiziert werden. Die Schnitte wurden 10 Minuten lang in PBS gelegt, welches 2% bovines Serumalbumin (BSA) und (mit Alexa Fluor^{®} 488-markiertes Phalloidin (Invitrogen, Katalog # A12379) in einer 1:40 Verdünnung enthielt. Danach wurden die Schnitte dreimal 10 Minuten lang mit PBS gewaschen und flach auf einen Objektträger gelegt. Ein Tropfen Pro Long DAPI, welcher Fixierungsmedium (Invitrogen, Kat # P36935) enthielt, wurde auf die Schnitte gegeben, die danach mit einem Deckgläschen abgedeckt wurden. Das Präparat wurde anschließend mit Nagellack versiegelt. Von den Schnitten wurden mit einem Fluoreszenzmikroskop (Keyence) in 40-facher Vergrößerung Aufnahmen gemacht. Die Figuren 7 und 8 zeigen repräsentative Ergebnisse aus Untersuchungen an jeweils vier Meerschweinchen.

Es zeigte sich in den unbehandelten Kontrollen (siehe Fig. 7A und Fig. 8A), dass es durch das akustische Trauma zu einem starken Absterben von äußeren Haarzellen kam. Dieses Absterben konnte durch eine intratympanale Verabreichung von lediglich insgesamt 0,105 mg 6-Fluor-9-methyl-β-carbolin verhindert werden (siehe Fig. 7B). Auch eine Verabreichung von 9-Methyl-β-carbolin konnte dem Absterben der äußeren Haarzellen entgegenwirken (siehe Fig. 8B), wobei es jedoch einer deutlichen höheren Dosis (insgesamt 0,2 mg 9-Methyl-β-carbolin) bedarf als beim erfindungsgemäßen 6-Fluor-9-methyl-β-carbolin. Folglich ist das erfindungsgemäße 6-Fluor-9-methyl-β-carbolin nicht nur zur Behandlung von Hörschädigungen geeignet, sondern auch effizienter als das 9-Methyl-β-carbolin aus dem Stand der Technik.

### Beispiel 7: akustisch evozierte Potenziale

Im Folgenden sollte die Wirkung von 6-Fluor-9-methyl-β-carbolin (6F9MβC) sowie von 9-Methyl-β-carbolin (9MβC) auf die durch ein akustisches Trauma induzierte Veränderung der Hörschwelle am Meerschweinchen-Modell untersucht werden.

Um ein akustisches Traum auszulösen wurden Meerschweinchen mit 60mg/kg Ketamin und 8mg/kg Xylazin betäubt. Dann wurde Bepantensalbe (Bayer) auf die Augen appliziert und das Tier in eine schalldichte Kammer gelegt. Ein kalibrierter Lautsprecher wurde direkt über dem Kopf der Tiers angebracht, wobei dieser so eingestellt war, dass er ein Halboktavband, um 8kHz zentriert, mit einer Lautstärke von 110 dB über eine Dauer von 30 Minuten emittierte.

Als Maß zur Beurteilung der Hörschwelle beim Meerschweinchen wurden akustisch evozierte Potenziale gemessen. Hierzu wurde die elektrophysiologische Aktivität der Nervenzellen im Hirnstamm, die die Impulse aus dem Innenohr weiterleiten, nach Beschallung des Ohrs mit 8, 11,3, 16, 22,6 und 32 kHz abgeleitet. Die Beschallung erfolgte aufsteigend von 0 dB bis 90 dB in Einzelschritten von 5 dB.

Die Messung erfolgte 3 Tage vor dem akustischen Trauma (D-3), 30 Minuten nach dem akustischen Trauma (D0) sowie 14 Tage nach dem akustischen Trauma (D14).

Je Tier wurde ein Ohr nicht behandelt und als intra-individuelle Kontrolle (hierin zum Teil auch mit "contr." abgekürzt) verwendet und das zweite Ohr mit 6-Fluor-9-methyl-β-carbolin behandelt. Hierzu wurde eine Stunde nach dem akustischen Traum eine Dosis von 0,12 mg 6-Fluor-9-methyl-β-carbolin (in einem Hydrogel als Vehikel) intratympanal verabreicht. 3 Tage nach dem akustischen Trauma erfolgte eine erneute intratympanale Verabreichung von 0,06 mg 6-Fluor-9-methyl-β-carbolin. Die Messungen wurden an vier Meerschweinchen durchgeführt (n = 4).

In Vergleichsexperimenten wurde festgestellt, dass eine bloße Gabe des Wirkstoff-Vehikels (also des Hydrogels) im Vergleich zu einem vollkommen unbehandelten Ohr keinen Effekt hatte.

Anschließend wurde aus den gemessenen Werten vor dem akustischen Trauma (D-3) und nach 14 Tagen (D14) die verbleibende Verschiebung der Hörschwelle (auch als permanente Verschiebung der Hörschwelle bezeichnet) bestimmt. Aus den gemessenen Werten direkt nach dem akustischen Trauma (D0) und nach 14 Tagen (D14) wurde zudem die Erholung von dem Trauma (D0 - D14) bestimmt.

Es zeigte sich, dass in Bezug zu der nach 14 Tagen noch verbleibenden Hörschädigung (siehe Fig. 9), die Verabreichung des 6-Fluor-9-methyl-β-carbolins (insgesamt 0,18 mg) zu einer deutlichen Verringerung der Hörschwelle im Vergleich zur unbehandelten Kontrolle führte. Besonders bei einer Schallfrequenz von 11,3 bzw. 16 kHz zeigte sich durch das erfindungsgemäße 6-Fluor-9-methyl-β-carbolin eine Verringerung der Hörschwelle um ca. 20 dB bzw. 30 dB.

Auch in Bezug zu der Erholung von dem akustischen Trauma (siehe Fig. 10) konnte durch die Verabreichung des 6-Fluor-9-methyl-β-carbolins (insgesamt 0,18 mg) die Erholung im Vergleich zur unbehandelten Kontrolle deutlich verbessert werden. So konnte bei Schallfrequenzen von 8, 11,3 bzw. 16 kHz eine um ca. 5 dB, 25 dB bzw. 20 dB verbesserte Erholung durch das erfindungsgemäße 6-Fluor-9-methyl-β-carbolin im Vergleich zum unbehandelten Ohr gezeigt werden.

Analog zu den oben durchgeführten Experimenten mit 6-Fluor-9-methyl-β-carbolin wurden auch entsprechende Experimente mit 9-Methyl-β-carbolin durchgeführt. Die Versuchsdurchführung war praktisch identisch, wobei jedoch natürlich 9-Methyl-β-carbolin an Stelle von 6-Fluor-9-methyl-β-carbolin. Die Gesamtdosis an 9-Methyl-β-carbolin betrug hierbei 0,2 mg bzw. 0,35 mg pro Ohr.

Auch das 9-Methyl-β-carbolin aus dem Stand der Technik förderte nachweislich die Erholung im Vergleich zur unbehandelten Kontrolle (siehe Fig. 11 und 12). Im Vergleich zum 6-Fluor-9-methyl-β-carbolin zeigte sich jedoch bei einer vergleichsweise bereits leicht erhöhten Dosis von 0,2 mg (siehe Fig. 11) lediglich eine Verbesserung der Erholung um ungefähr 5 dB bis maximal 10 dB. Auch bei einer weiter erhöhten Dosis von 0,35 mg 9-Methyl-β-carbolin (doppelt so hoch wie beim 6-Fluor-9-methyl-β-carbolin) zeigte sich lediglich eine Verbesserung um ungefähr 10 dB (siehe Fig. 12).

### Beispiel 8: Abbau in der Leber zu toxischen Abbauprodukten

Im Folgenden sollte untersucht werden, ob das erfindungsgemäße 6-Fluor-9-methyl-β-carbolin (6F9MβC) in der Leber zu bekannten toxischen Abbauprodukten metabolisiert wird.

Für β-Carboline ist seit langem bekannt, dass diese in einer von N-Methyltransferasen katalysierten Reaktion zu methylierten β-Carbolinium-Kationen umgesetzt werden. Diese haben einige toxikologische Eigenschaften mit dem 1-Methyl-4-phenylpyridinium (MPP+, auch als Parkinson Neurotoxin bekannt) gemeinsam, da sie u.a. die mitochondriale Respiration hemmen können und neurotoxisch wirken. Besonders toxisch sind hierbei die dimetyhlierten β-Carbolinium-Kationen, d.h. die 2,9-Dimethyl-β-carbolinium-Kationen. Diese werden durch sequentielle Methylierung an Position 2 und 9 gebildet. Daher sollte im Folgenden untersucht werden, ob das erfindungsgemäße 6-Fluor-9-methyl-β-carbolin (6F9MβC) in der Leber zu den entsprechenden Toxinen 6-Fluor-2,9-dimethyl-β-carbolinium-Kation (direkt abgeleitet vom 6F9MβC) bzw. 2,9-Dimethyl-β-carbolinium-Kation (abgeleitet vom 9MβC) umgesetzt wird.

Um diese Substanzen mittels HPLC identifizieren zu können, wurden sowohl das 6-Fluor-2,9-dimethyl-β-carbolinium-Kation (6F29DMβC) als auch das 2,9-Dimethyl-β-carbolinium-Kation (29DMβC) vor Ort synthetisiert und die jeweiligen Retentionszeiten durch Referenz-HPLC-Messungen bestimmt. Ebenfalls wurde eine Referenzmessung mit reinem 6-Fluor-9-methyl-β-carbolin (6F9MβC) durchgeführt.

Um den Abbauprozess in der Leber zu simulieren wurden verschiedene Konzentrationen (1 µM, 10 µM und 100 µM) an 6-Fluor-9-methyl-β-carbolin (6F9MβC) für eine Stunde unter physiologischen Bedingungen (37°C in physiologsicher Phosphat-gepufferter Salzlösung, PBS) mit homogenisierter Leber der Ratte inkubiert. Anschließend erfolgte eine HPLC-Messung eines filtrierten Extrakts.

Die Charakterisierung der jeweiligen Substanzen erfolgte über Hochleistungsflüssigkeitschromatographie (HPLC, z.T. auch als Hochdruckflüssigkeitschromatographie bezeichnet). Bei der HPLC bestand der Eluent zu 45 % aus 90%igem Acetonitril und zu 55 % aus Ammoniumazetat (40 mM, pH <5). Die Flussrate betrug 0,4 mL/min und das Injektionsvolumen war 10 µL. Jede Probe wurde mit 60 % Methanol verdünnt, sofern das Signal zu stark war. So musste z.B. die Probe aus dem Leberhomogenat vor der Messung 1:40 verdünnt werden, um im dynamischen Bereich des Analyseverfahrens zu liegen.

Für das unbehandelte 6-Fluor-9-methyl-β-carbolin (6F9MβC) wurde eine Retentionszeit von 19,0 Minuten gemessen (siehe Fig. 13A), während die Referenzmessungen für 6-Fluor-2,9-dimethyl-β-carboliniumion (6F29DMβC) eine Retentionszeit von 10,1 Minuten (siehe Fig. 13C) und für 2,9-Dimethyl-β-carboliniumion (29DMβC) eine Retentionszeit von 9,1 Minuten (siehe Fig. 13D) zeigten. Alle drei Referenzproben zeigten also deutlich voneinander trennbare Retentionszeiten. Das mit Leberhomogenaten der Ratte inkubierte 6-Fluor-9-methyl-β-carbolin (100 µM) zeigte zwar den Peak bei 19,0 Minuten (also von 6F9MβC), jedoch keine Peaks für 6-Fluor-2,9-dimethyl-β-carboliniumion (6F29DMβC) oder 2,9-Dimethyl-β-carboliniumion (29DMβC). Die ebenfalls getesteten niedrigeren Konzentrationen von 6-Fluor-9-methyl-β-carbolin (1 µM und 10 µm) zeigten identische Resultate.

Es kann gefolgert werden, dass das erfindungsgemäße 6-Fluor-9-methyl-β-carbolin unter den gegebenen Bedingungen erfreulicherweise nicht zu bekannten toxischen Abbauprodukten metabolisiert wird.

Im Folgenden sei noch kurz die Synthese für die Referenzkationen 6-Fluor-2,9-dimethyl-β-carboliniumion (6F29DMβC) und 2,9-Dimethyl-β-carboliniumion (29DMβC) beschrieben.

Zur Synthese des 6F29DMβC werden zu einer Lösung von 50 mg (d.h. 0,25 mmol) 6-Fluor-9-methyl-β-carbolin (freie Base) in 3 mL Acetonitril 200 mg (d.h. 1,4 mmol) Methyliodid gegeben. Die Reaktionsmischung wird unter Stickstoff für 24h bei Raumtemperatur gerührt. Dann werden die Lösungsmittel im Abzug unter einem Stickstoffstrom abgeblasen. Der gelbe, fluoreszierende Rückstand wird aus wenig Acetonitril umkristallisiert. Ausbeute: 53 mg (d.h. 15 mmol) 60%.

Zur Synthese des 29DMβC werden zu einer Lösung von 50 mg (d.h. 0,27 mmol) 9-Methyl-β-carbolin (freie Base) in 3 mL Acetonitril werden 200 mg (d.h. 1,4 mmol) Methyliodid gegeben. Die Reaktionsmischung wird unter Stickstoff für 24h bei Raumtemperatur gerührt. Dann werden die Lösungsmittel im Abzug unter einem Stickstoffstrom abgeblasen. Der gelbe, fluoreszierende Rückstand wird aus wenig Acetonitril umkristallisiert, abfiltriert und im Vakuum getrocknet. Ausbeute: 43 mg (d.h. 13 mmol) 48%.

### Beispiel 9: 6-Fluor-9-methyl-β-carbolin verbessert Lernen und Gedächtnis

Die dem Lernen und Gedächtnis zugrunde liegenden Mechanismen sind äußerst komplex. Sie können auf molekularer und zellulärer Ebene, auf der Ebene der Systembiologie und der der Psychologie betrachtet werden. Einer der Schlüsselprozesse für die Gedächtnisbildung auf molekularer Ebene ist die Aktivierung des cAMP response element binding proteins (CREB). Diese führt zur Neubildung von Synapsen, ein Vorgang, an den die Gedächtnisbildung gekoppelt ist. Dieser Vorgang wird durch viele Faktoren reguliert. Eine positive Verstärkung wird durch den brain derived neurotrophic factor (BDNF) und seinen Rezeptor TRKB induziert. Daher sollte auf zellulärer Ebene untersucht werden, ob das erfindungsgemäße 6-Fluor-9-Methyl-β-carbolin (6F9MβC)

Wir haben in unseren Untersuchungen auf zellulärer Ebene gefunden, dass das 6-Fluor-9-Methyl-β-carbolin (6F9MβC) die Konzentration von CREB um den Faktor 8 erhöht und die Konzentration von BDNF bis zu dreifach erhöht. Diese Befunde legen nahe, dass 6F9MβC das Lernen und Gedächtnis fördert. Diese Hypothese wurde im folgenden Experiment geprüft.

In einem achtarmigen Labyrinth wurde die Wirkung von 6F9MβC auf das Lernverhalten getestet. 5 Monate alte männliche Ratten wurden in täglichen Sitzungen 10 Tage lang an den Experimentator gewöhnt. Danach konnten sie in mehreren Sitzungen 2 Tage lang das Labyrinth erkunden und lernten auch, dass sie dort eine Futterbelohnung erhalten. Dann folgte eine Futterreduzierung bis das Körpergewicht um 10% des Ausgangsgewichts reduziert war. Danach wurde an jedem Ende der 8 Arme des Labyrinths Futter platziert und eine Kamera installiert, die jede Bewegung des Tiers aufzeichnete. Am ersten Versuchstag wurde die Ratte ohne vorhergehende Injektion auf die Plattform in der Mitte des Labyrinths gesetzt. Am nächsten Tag wurden der Ratte vom Experimentator 2mg/kg Körpergewicht 6-Fluor-9-methyl-β-carbolin (6F9MβC) als freie Base gelöst in physiologischer Kochsalzlösung intraperitoneal appliziert. 2 Stunden danach wurde das Tier auf die Plattform in der Mitte des Labyrinths gesetzt von der aus sie Zugang zu allen Armen hatte. Diese Prozedur wurde täglich wiederholt bis das Tier maximal einen Fehler machte, d.h. in einen Arm ging, den es bereits zuvor in der betreffenden Sitzung betreten hatte. Am Ende der Sitzung mussten alle 8 Arme besucht worden sein.

Untersucht wurden 12 Ratten, denen 6F9MβC appliziert worden war und eine Kontrollgruppe von 12 Ratten, die nur physiologische Kochsalzlösung bekommen hatte. Die Verum-Gruppe (hat 6F9MβC erhalten) erreichte das Kriterium nach 6 Tagen und die Kontrollgruppe nach 14 Tagen. Der Unterschied wurde mit der ANOVA für wiederholte Messungen geprüft und war signifikant (p<0,01). Diese Ergebnisse belegen eindeutig, dass 6F9MβC Lernprozesse beschleunigt und das Gedächtnis verbessert. Wir konnten also unsere Hypothese verifizieren.

Damit ist gezeigt, dass der Wirkstoff 6F9MβC nicht nur bei Erkrankungen des Innenohrs wirksam ist, sondern auch ein komplexes Verhalten wie das Lernen und Gedächtnis günstig beeinflusst.

### Literatur:

1.) Application of high-performance liquid chromatography based measurements of lipophilicity to model biological distribution. Valkó K Journal of Chromatography A 2004;1037 (1-2): 299-310.
2.) The levels of norharman are high enough after smoking to affect monoamineoxidase B in platelets. Rommelspacher et al., Eu J Pharmacol 2002;441 (1-2): 115-125.

## Patentansprüche

1. Verbindung der allgemeinen Formel (I) 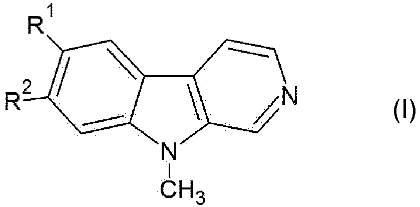 wobei
R¹ = -F und R² = -H oder -F ist, oder
R¹ = -H und R² = -F ist;
sowie pharmakologisch verträgliche Salze, Solvate und Hydrate der vorgenannten Verbindungen.

2. Verbindung gemäß Anspruch 1, wobei R¹ = -F und R² = -H ist.

3. Verfahren zur Herstellung einer Verbindung gemäß Anspruch 1, umfassend die folgenden Schritte:
a) Reaktion der Ausgangsverbindung der allgemeinen Formel (II) unter Zugabe von Glyoxalsäurehydrat der Formel (III) und einer Base zu einer Verbindung der allgemeinen Formel (IV) 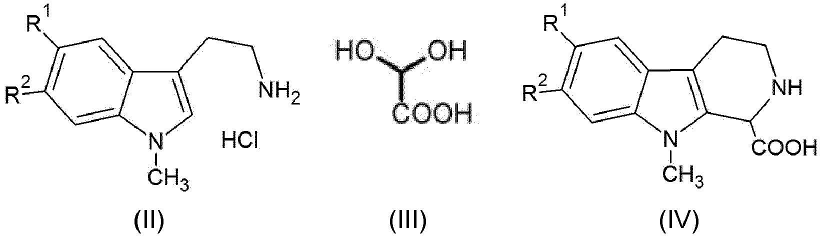
b) Decarboxylierung der Verbindung der allgemeinen Formel (IV) unter Zugabe einer Säure und unter Erhitzen zu einer Verbindung der allgemeinen Formel (V) 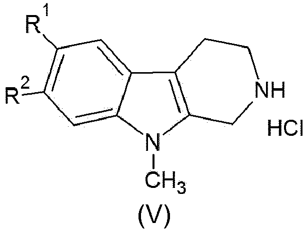
c) Aromatisierung der Verbindung der allgemeinen Formel (V) zu einer Verbindung der allgemeinen Formel (I) unter Zugabe eines Katalysators;
wobei
R¹ = -F und R² = -H oder -F ist, oder
R¹ = -H und R² = -F ist.

4. Verfahren gemäß Anspruch 3, **dadurch gekennzeichnet, dass** es sich bei dem in Schritt c) verwendeten Katalysator um Pd / C handelt.

5. Verbindung gemäß Anspruch 1 oder 2 zur Verwendung als Medikament.

6. Verbindung gemäß Anspruch 1 oder 2 zur Verwendung in der Behandlung und/oder Schädigungen des Innenohres.

7. Verbindung zur Verwendung gemäß Anspruch 6, **dadurch gekennzeichnet, dass** es sich bei den Erkrankungen und/oder Schädigungen des Innenohres um akute Erkrankungen des Innenohres handelt, ausgewählt aus der Gruppe umfassend Knalltrauma, Explosionstrauma des Ohres, Hörsturz und Insertionstrauma.

8. Verbindung zur Verwendung gemäß Anspruch 6, **dadurch gekennzeichnet, dass** es sich bei den Erkrankungen und/oder Schädigungen des Innenohres um chronische Erkrankungen des Innenohres handelt, ausgewählt aus der Gruppe umfassend chronisches Schalltrauma, Tinnitus und Presbyakusis.

9. Verbindung zur Verwendung gemäß einem der Ansprüche 6 bis 8, **dadurch gekennzeichnet, dass** die Schädigung des Innenohrs durch ototoxische Substanzen bedingt ist.

10. Pharmazeutische Zusammensetzung in Form von Tropfen, Salben, Sprays, Liposomen, Gelen, Emulsionen oder Injektionslösungen enthaltend mindestens eine Verbindung gemäß Anspruch 1.

## Claims

1. Compounds of general formula (I) 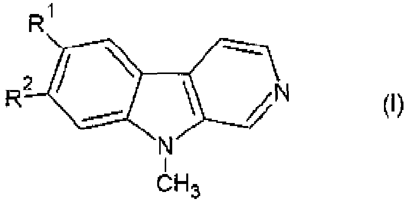 wherein
R¹ is -F and R² is -H or -F, or
R¹ is -H and R² is -F;
as well as pharmacologically acceptable salts, solvates and hydrates of the aforementioned compounds.

2. Compound according to claim 1, wherein R¹ is -F and R² is -H.

3. Process for preparing a compound according to claim 1, comprising the following steps:
a) Reaction of starting material of general formula (II) under addition of glyoxylic acid hydrate of formula (III) and a base to a compound of general formula (IV) 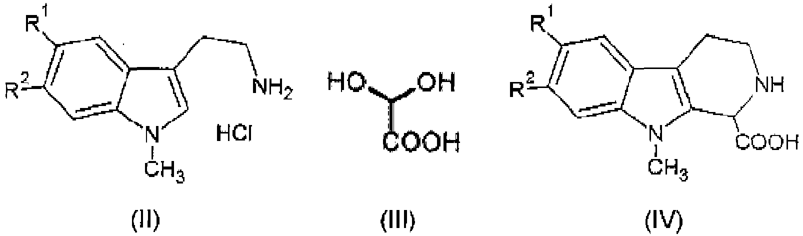
b) Decarboxylation of the compound of general formula (IV) under addition of an acid and under heating to a compound of general formula (V) 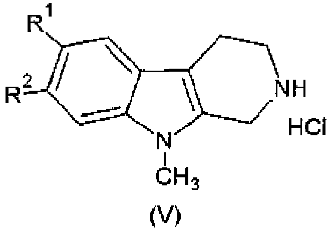
c) Aromatization of the compound of general formula (V) to a compound of general formula (I) under addition of a catalyst
wherein
R¹ is -F and R² is -H or -F, or
R¹ is -H and R² is -F.

4. Process according to claim 3, **characterized in that** the catalyst used in step c) is Pd/C.

5. Compound according to claim 1 or 2 for use as a medicament.

6. Compound according to claim 1 or 2 for use in treatment of diseases and/or damages of the inner ear.

7. Compound for use according to claim 6, **characterized in that** diseases and/or damages of the inner ear are acute diseases of the inner ear, selected from the group comprising blast trauma, blast injury of the ear, hearing loss and insertion trauma,

8. Compound for use according to claim 6, **characterized in that** diseases and/or damages of the inner ear are chronic diseases of the inner ear, selected from the group comprising chronic acoustic trauma, tinnitus and presbyacusis.

9. Compound for use according to any one of clams 6 - 8, **characterized in that** damage of the inner ear is caused by ototoxic substances.

10. Pharmaceutical composition in form of droplets, ointments, sprays, liposomes, gels, emulsions or injection solutions containing at least one compound according to claim 1.

## Revendications

1. Un composé de formule générale (I) 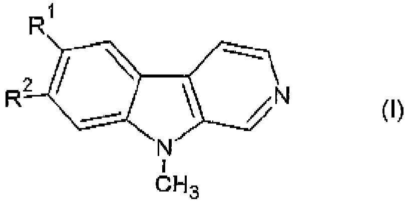 où
R¹ = -F et R² = -H ou -F, ou
R¹ = -H et R² = -F ;
ainsi que des sels pharmacologiquement acceptables, des solvates et des hydrates du composé précité.

2. Le composé selon la revendication 1, où R¹ = -F et R² = -H.

3. Un procédé pour la préparation d'un composé selon la revendication 1, comprenant les étapes suivantes:
a) faire réagir le composé de départ de formule générale (II) avec l'hydrate d'acide carboxylique de formule générale (III) et une base pour donner le composé de formule générale (IV) 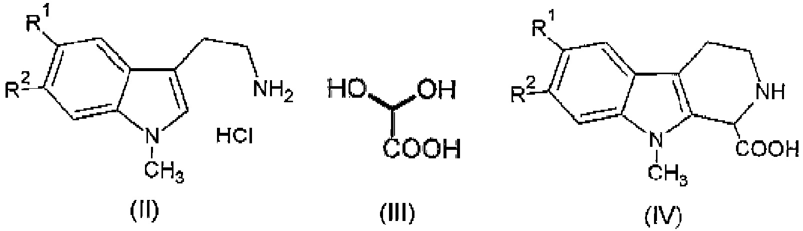
b) décarboxylation du composé de formule générale (IV) en présence d'un acide et sous chauffage pour donner le composé de formule générale (V) 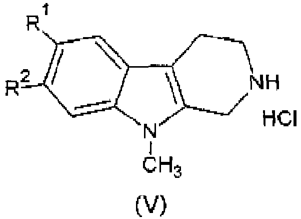
c) aromatisation du composé de formule générale (V) sous l'addition d'un catalyseur pour donner le composé de formule générale (I).
où
R¹ = -F et R² = -H ou -F, ou
R¹ = -H et R² = -F.

4. Le procédé selon la revendication 3, **caractérisé en ce que** le catalyseur utilisé à l'étape c) est Pd/C.

5. Le composé selon la revendication 1 ou 2 destiné à être utilisé comme médicament.

6. Le composé selon la revendication 1 ou 2 destiné à être utilisé dans le traitement des maladies et/ou des troubles de l'oreille interne.

7. Le composé destiné à être utilisé selon la revendication 6, **caractérisé en ce que**, les maladies et/ou les troubles de l'oreille interne sont des maladies aigues de l'oreille interne sélectionnées parmi le groupe comprenant le traumatisme sonore, le traumatisme de l'oreille par explosion, la perte auditive et le traumatisme par insertion.

8. Le composé destiné à être utilisé selon la revendication 6, **caractérisé en ce que**, les maladies et/ou les troubles de l'oreille interne sont des maladies chroniques de l'oreille interne sélectionnées parmi le groupe comprenant le traumatisme chronique acousticiue, le tinnitus et la presbyacousie.

9. Le composé destiné à être utilisé selon une quelconque des revendications 6 à 8, **caractérisé en ce que**, le trouble de l'oreille interne est provoqué par des substances ototoxiques.

10. Composition pharmaceutique sous forme de gouttelettes, onguents, sprays, liposomes, gels, émulsions ou solutions à injecter contenant au moins un composé selon la revendication 1.
